(19) Europäisches Patentamt European Patent Office Office européen des brevets

(11) **EP 4 675 276 A1**

(12) **EUROPEAN PATENT APPLICATION**
published in accordance with Art. 153(4) EPC

(43) Date of publication:
07.01.2026 Bulletin 2026/02

(21) Application number: 24763936.2

(22) Date of filing: 27.02.2024

(51) International Patent Classification (IPC):
*G01N 33/53* (2006.01)     *C07K 14/435* (2006.01)
*C12P 21/02* (2006.01)     *G01N 33/72* (2006.01)
*G01N 33/543* (2006.01)

(52) Cooperative Patent Classification (CPC):
C07K 14/435; C12P 21/02; G01N 33/53;
G01N 33/543; G01N 33/72

(86) International application number:
PCT/JP2024/007134

(87) International publication number:
WO 2024/181457 (06.09.2024 Gazette 2024/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.02.2023 JP 2023028613
05.07.2023 JP 2023110967

(71) Applicant: EIKEN KAGAKU KABUSHIKI KAISHA
Tokyo 110-8408 (JP)

(72) Inventors:
• MAKINODAN, Mitsuru
Shimotsuga-gun, Tochigi 329-0114 (JP)
• KAWAGUCHI, Kouhei
Shimotsuga-gun, Tochigi 329-0114 (JP)
• YUI, Megumi
Shimotsuga-gun, Tochigi 329-0114 (JP)
• FUJII, Koichi
Shimotsuga-gun, Tochigi 329-0114 (JP)
• KOZUKA, Naoyuki
Shimotsuga-gun, Tochigi 329-0114 (JP)

(74) Representative: Kuhnen & Wacker
Patent- und Rechtsanwaltsbüro PartG mbB
Prinz-Ludwig-Straße 40A
85354 Freising (DE)

Remarks:
The complete document including Reference Table(s) and the Sequence Listing(s) can be downloaded from the EPO website

(54) **METHOD FOR IMMUNOLOGICALLY MEASURING HEMOGLOBIN USING DEGLYCOSYLATED HAPTOGLOBIN**

(57) An immunological measurement method for hemoglobin, comprising a step of performing the immunological measurement using an anti-hemoglobin antibody under coexistence of the hemoglobin and haptoglobin, the haptoglobin having a characteristic (1) below: (1) at least a portion of an N-linked glycan is deleted. The present invention further provides a method for suppressing change in measurement values of hemoglobin, a method and a solution for stabilizing hemoglobin, a hemoglobin measurement kit, and the haptoglobin and a method for producing the same. According to the present invention, hemoglobin can be measured while suppressing haptoglobin-associated change in hemoglobin measurement values.

[FIG. 8]

(a) Equine Hpt (without enzyme treatment)
(b) Equine Hpt (ENGase treatment)
(c) Equine Hpt (PNGase treatment)

## Description

### Technical Field

The present invention relates to an immunological measurement method for hemoglobin, and particularly to an immunological measurement method using deglycosylated haptoglobin. The present invention further relates to a method for suppressing change in measurement values of hemoglobin, a method and a solution for stabilizing hemoglobin, a hemoglobin measurement kit, and the haptoglobin and a method for producing the same.

### Background Art

Detection of blood contained in specimens such as feces, urine, and saliva is useful for diagnosing many diseases. For example, fecal occult blood tests, which detect blood in feces, are used in colon cancer screening. As a method for detecting occult blood, an immunological scheme is known, which uses anti-hemoglobin antibodies to detect hemoglobin (which may be written as Hb, hereinafter) contained in occult blood in specimens such as feces.

A specimen to be used for an occult blood test is usually collected by the subject in a container containing a preservative solution and sent to a testing institution such as a hospital. In many cases, the preservative solution containing the specimen is stored for several days until it is actually used for testing, and during that time it is often placed under high temperatures. Hemoglobin is unstable in solution and is particularly prone to denaturation or decomposition under high temperature conditions. If the structure of the epitope or its surrounding area changes due to the denaturation or decomposition of hemoglobin, the antibody will no longer be able to recognize the hemoglobin, and the accuracy of hemoglobin detection by the immunological scheme will therefore decrease.

Hence, to stabilize hemoglobin, methods of adding haptoglobin (which may be written as Hpt, hereinafter) to the preservation solution for a specimen are used (e.g., Patent Document 1 and Patent Document 2). Haptoglobin is a protein that plays a role in recovering hemoglobin released into the blood due to hemolysis of red blood cells. It is known that haptoglobin quickly and irreversibly binds to hemoglobin to form a stable hemoglobin-haptoglobin complex (which may be written as Hb-Hpt, hereinafter). By preliminarily adding haptoglobin to a preservation solution or the like, when the specimen is added to the preservation solution or the like, the hemoglobin contained in the specimen forms a hemoglobin-haptoglobin complex and is stabilized.

It is, however, known that changes may be recognized in the measured values of hemoglobin due to haptoglobin. (Patent Document 1 and Patent Document 3).

### Prior Art Documents

### Patent Documents

    Patent Document 1: JP10-132824A
    Patent Document 2: WO2011/058958
    Patent Document 3: WO2020/066722

### Summary of the Invention

### Problems to be solved by the Invention

The present invention has been made in consideration of the above problems, and objects of the present invention include providing an immunological measurement method, a method and a solution for stabilizing hemoglobin, an immunological measurement kit for hemoglobin, and haptoglobin that allow hemoglobin to be measured while suppressing change in the hemoglobin measurement values caused by haptoglobin.

### Means for solving the Problems

As a result of research to solve the above problems, the present inventors have found that by using haptoglobin whose glycan is arranged in a specific configuration, it is possible to suppress change in measurement values of hemoglobin regardless of the form of free hemoglobin or hemoglobin-haptoglobin complex, and have thus completed the present invention. Specifically, the present invention is as follows.

[1] An immunological measurement method for hemoglobin, comprising

a step of performing the immunological measurement using an anti-hemoglobin antibody under coexistence of the hemoglobin and haptoglobin,
the haptoglobin having a characteristic (1) below:

(1) at least a portion of an N-linked glycan is deleted.

[2] The immunological measurement method according to [1], wherein the haptoglobin further has a characteristic (2) below:
(2) when 3.1 pmol of hemoglobin is stored at 37°C for 7 days in 500 μL of a buffer solution containing 3.1 pmol of the haptoglobin, a residual ratio of the hemoglobin is 70% or more.
[3] The immunological measurement method according to [1] or [2], wherein the characteristic (1) is a characteristic (1a) below:
(1a) a difference between a molecular weight of a β-chain in the haptoglobin treated with peptide-N-glycosidase (PNGase) and a molecular weight of a β-chain in an untreated haptoglobin is 6,500 or less.
[4] The immunological measurement method according to any one of [1] to [3], wherein the haptoglobin has no N-linked glycan.
[5] The immunological measurement method according to any one of [1] to [3], wherein the N-linked glycan present in the haptoglobin from which at least a portion of the N-linked glycan is deleted is one N-acetylglucosamine residue or a glycan in which one fucose residue is bound to one N-acetylglucosamine residue.
[6] The immunological measurement method according to any one of [1] to [5], wherein the hemoglobin is contained in feces, blood, serum, plasma, urine, sputum, or saliva.
[7] The immunological measurement method according to any one of [1] to [6], wherein at least one type of the anti-hemoglobin antibody is supported on an insoluble carrier.
[8] The immunological measurement method according to [7], wherein the insoluble carrier is an insoluble particle.
[9] The immunological measurement method according to any one of [1] to [8], wherein the immunological measurement method is an immune agglutination method.
[10] A method for suppressing, in immunological measurement of hemoglobin, change in measurement values of the hemoglobin, comprising

a step of performing the immunological measurement using an anti-hemoglobin antibody under coexistence of the hemoglobin and haptoglobin,
the method using as the haptoglobin a haptoglobin having a characteristic (1) below:

(1) at least a portion of an N-linked glycan is deleted.

[11] The method for suppressing change in measurement values of hemoglobin according to [10], wherein the haptoglobin further has a characteristic (2) below:
(2) when 3.1 pmol of hemoglobin is stored at 37°C for 7 days in 500 μL of a buffer solution containing 3.1 pmol of the haptoglobin, a residual ratio of the hemoglobin is 70% or more.
[12] A method for stabilizing hemoglobin, comprising a step of allowing hemoglobin to coexist with haptoglobin having a characteristic (1) below:

(1) at least a portion of an N-linked glycan is deleted.

[13] The method for stabilizing hemoglobin according to [12], wherein the haptoglobin further has a characteristic (2) below:
(2) when 3.1 pmol of hemoglobin is stored at 37°C for 7 days in 500 μL of a buffer solution containing 3.1 pmol of the haptoglobin, a residual ratio of the hemoglobin is 70% or more.
[14] The method for stabilizing hemoglobin according to [12] or [13], wherein the haptoglobin has no N-linked glycan.
[15] The method for stabilizing hemoglobin according to (12) or (13), wherein the N-linked glycan present in the haptoglobin from which at least a portion of the N-linked glycan is deleted is one N-acetylglucosamine residue or a glycan in which one fucose residue is bound to one N-acetylglucosamine residue.
[16] A solution for stabilizing hemoglobin, comprising haptoglobin having a characteristic (1) below:

(1) at least a portion of an N-linked glycan is deleted.

[17] The solution for stabilizing hemoglobin according to [16], wherein the haptoglobin further has a characteristic (2) below:

(2) when 3.1 pmol of hemoglobin is stored at 37°C for 7 days in 500 μL of a buffer solution containing 3.1 pmol of the haptoglobin, a residual ratio of the hemoglobin is 70% or more.

[18] The solution for stabilizing hemoglobin according to [16] or [17], wherein the characteristic (1) is a characteristic (1a) below:

(1a) a difference between a molecular weight of a β-chain in the haptoglobin treated with peptide-N-glycosidase (PNGase) and a molecular weight of a β-chain in an untreated haptoglobin is 6,500 or less.

[19] The solution for stabilizing hemoglobin according to any one of (16) to [18], wherein the haptoglobin has no N-linked glycan.

[20] The solution for stabilizing hemoglobin according to any one of (16) to [19], wherein the haptoglobin is haptoglobin that is naturally-derived and treated with peptide-N-glycosidase (PNGase).

[21] The solution for stabilizing hemoglobin according to any one of [16] to [18], wherein the N-linked glycan present in the haptoglobin from which at least a portion of the N-linked glycan is deleted is one N-acetylglucosamine residue or a glycan in which one fucose residue is bound to one N-acetylglucosamine residue.

[22] The solution for stabilizing hemoglobin according to any one of (16) to (18) and [21], wherein the haptoglobin is haptoglobin that is naturally-derived and treated with endo-β-N-acetylglucosaminidase (ENGase).

[23] The solution for stabilizing hemoglobin according to any one of [16] to [22], wherein the hemoglobin is contained in feces, blood, serum, plasma, urine, sputum, or saliva.

[24] A hemoglobin measurement kit for immunologically measuring hemoglobin, comprising:

the solution for stabilizing hemoglobin according to any one of (16) to (23) ; and
a reagent containing an anti-hemoglobin antibody.

[25] The hemoglobin measurement kit according to [24], wherein at least one type of the anti-hemoglobin antibody is supported on an insoluble carrier.

[26] The hemoglobin measurement kit according to [25], wherein the insoluble carrier is an insoluble particle.

[27] The hemoglobin measurement kit according to any one of (24) to [26], wherein the reagent is a reagent for an immune agglutination method.

[28] Haptoglobin having characteristics (1) and (2) below:

(1) at least a portion of an N-linked glycan is deleted; and
(2) when 3.1 pmol of hemoglobin is stored at 37°C for 7 days in 500 μL of a buffer solution containing 3.1 pmol of the haptoglobin, a residual ratio of the hemoglobin is 70% or more.

[29] The haptoglobin according to [28], wherein the characteristic (1) is a characteristic (1a) below:
(1a) a difference between a molecular weight of a β-chain in the haptoglobin treated with peptide-N-glycosidase (PNGase) and a molecular weight of a β-chain in an untreated haptoglobin is 6,500 or less.

[30] The haptoglobin according to [28] or [29], wherein the haptoglobin has no N-linked glycan.

[31] The haptoglobin according to any one of [28] to [30], wherein the haptoglobin is haptoglobin that is naturally-derived and treated with peptide-N-glycosidase (PNGase).

[32] The haptoglobin according to [28] or [29], wherein the N-linked glycan present in the haptoglobin from which at least a portion of the N-linked glycan is deleted is one N-acetylglucosamine residue or a glycan in which one fucose residue is bound to one N-acetylglucosamine residue.

[33] The haptoglobin according to any one of [28], [29], and [32], wherein the haptoglobin is haptoglobin that is naturally-derived and treated with endo-β-N-acetylglucosaminidase (ENGase).

[34] Haptoglobin having an N-linked glycan, the glycan of the N-linked glycan being one N-acetylglucosamine residue or only a glycan in which one fucose residue is bound to one N-acetylglucosamine residue.

[35] A method for producing haptoglobin, comprising a step of subjecting naturally-derived haptoglobin to a glycosidase treatment, and satisfying (a) and (b) below:

(a) the naturally-derived haptoglobin is not subjected to a heat shock of 90°C or higher prior to the glycosidase treatment; and
(b) SDS is not allowed to coexist during the glycosidase treatment.

[36] The method for producing haptoglobin according to [35], wherein the glycosidase is peptide-N-glycosidase (PNGase).

[37] The method for producing haptoglobin according to [36], wherein the peptide-N-glycosidase is PNGase F.

[38] The method for producing haptoglobin according to [35], wherein the glycosidase is endo-β-N-acetylglucosaminidase (ENGase).

[39] The method for producing haptoglobin according to [38], wherein the endo-β-N-acetylglucosaminidase is Endo F2.

**Advantageous Effect of the Invention**

According to the haptoglobin-containing storage solution for hemoglobin, the hemoglobin measurement kit, the immunological measurement method, and the haptoglobin of the present invention, change in the measured values of hemoglobin caused by haptoglobin can be suppressed.

**Brief Description of the Drawings**

[FIG. 1] FIG. 1 is a set of schematic diagrams of N-linked glycans.

[FIG. 2] FIG. 2 illustrates the amino-acid sequence of type 2 human preprohaptoglobin (SEQ ID NO: 1). In FIGS. 2 to 5, the underlined portions represent consensus sequences specific to N-linked glycan addition, and the bolded portions represent asparagine residues that undergo N-linked glycan addition.

[FIG. 3] FIG. 3 illustrates the amino-acid sequence of type 1 human preprohaptoglobin (SEQ ID NO: 2).

[FIG. 4] FIG. 4 illustrates the amino-acid sequence of equine preprohaptoglobin (SEQ ID NO: 3).

[FIG. 5] FIG. 5 illustrates the amino-acid sequence of porcine preprohaptoglobin (SEQ ID NO: 4).

[FIG. 6] FIG. 6 is a graph illustrating the results of measuring mixture liquids (specimens) of free hemoglobin (free-Hb)/hemoglobin-haptoglobin (Hb-Hp) complex using the immunoagglutination assay reagent prepared in Reference Example 4 and a commercially available colorimetric assay reagent.

[FIG. 7] FIG. 7 is a set of photographs showing electrophoresis (SDS-PAGE) images confirming the results of deglycosylation for porcine Hpt, equine Hpt, and human Hpt.

[FIG. 8] FIG. 8 is a set of diagrams illustrating MALDI-TOF mass spectra confirming the results of deglycosylation for equine Hpt.

[FIG. 9] FIG. 9 is a set of diagrams illustrating MALDI-TOF mass spectra confirming the presence or absence of change in molecular weight due to reduced PNGase treatment for deglycosylated equine Hpt (dHpt) and untreated equine Hpt.

[FIG. 10] FIG. 10 is a graph illustrating the results of forming complexes with free-Hb at various concentrations for various Hpts and equine dHpt and measuring the amount of remaining free-Hb.

**Embodiments for Carrying out the Invention**

Hereinafter, one or more embodiments of the present invention will be described.

[Terminology]

(Hemoglobin)

Hemoglobin, which is a protein contained in red blood cells in the living organisms, has the property of binding to oxygen molecules and is involved in the transport of oxygen.

Hemoglobin has a tetrameric structure [$\alpha_2\beta_2$] with two types of subunits, consisting of two $\alpha$ and two $\beta$ chains (subunits), each made of 141 and 146 amino acids, respectively. The molecular weight of the $\alpha$ chain is approximately 15,500, the molecular weight of the $\beta$ chain is approximately 17,000, and the molecular weight of the entire hemoglobin is approximately 64,500.

(Haptoglobin)

Haptoglobin, which is a type of glycoprotein, specifically binds to hemoglobin to form a hemoglobin-haptoglobin complex.

The origin of the haptoglobin used in the present embodiment is not particularly limited, provided that it forms a hemoglobin-haptoglobin complex by complexing with hemoglobin. Since the binding between hemoglobin and haptoglobin has low species specificity, haptoglobin originated from a wide range of species can be used. When the hemoglobin in a specimen is human hemoglobin, haptoglobin originated from humans as well as from animals such as horses, pigs, monkeys, dogs, and cows can be used. Recombinant haptoglobin can also be used. Haptoglobin does not necessarily have to be highly purified.

There are three serotypes of human haptoglobin: type 1-1, type 2-1, and type 2-2. Haptoglobin is composed of an $\alpha$ chain and a $\beta$ chain, and in humans there are two types of $\alpha$ chains ($\alpha 1$ and $\alpha 2$) and one type of $\beta$ chain.

Human type 1-1, which has the simplest structure, is composed of two $\alpha 1$ chains and two $\beta$ chains and can be written as $[\alpha 1\beta]_2$. In human type 1-1, the $\alpha 1$ chain and the $\beta$ chain are linked by S-S bonds, and the $\alpha 1$ chains are linked to each other by S-S bonds. On the other hand, human type 2-1 can be written as $[\alpha 1\beta]_2[\alpha 2\beta]_n$, where n=0, 1, 2, ..., and human type 2-2 can be written as $[\alpha 2\beta]_m$, where m=3, 4, 5, ....

Haptoglobin is known to have an asparagine residue in the $\beta$ chain modified with a glycan (N-linked glycan).

The molecular weights are approximately 10,000 for the $\alpha 1$ chain, approximately 18,000 for the $\alpha 2$ chain, and approximately 39,000 for the $\beta$ chain (modified with a glycan). The molecular weight of the entire haptoglobin is, for example, approximately 98,000 for type 1-1.

Haptoglobin is translated as preprohaptoglobin having a signal sequence, an $\alpha$ chain, and a $\beta$ chain, the signal sequence is cleaved and removed, and further the main chain (peptide bond) between the $\alpha$ chain and the $\beta$ chain is cleaved and a disulfide bond is formed, forming a dimer of the $\alpha$ chain and the $\beta$ chain, forming the basic structure of the haptoglobin molecule, and when the above dimers associate, various types of haptoglobin molecules (such as $[\alpha 1\beta]_2$, $[\alpha 1\beta]_2[\alpha 2\beta]_n$, and $[\alpha 2\beta]_m$) are formed.

Haptoglobin is also known in other mammals, birds, etc., and for example, haptoglobin from horses, pigs, etc. is known to have a structure like human type 1-1 (i.e., $[\alpha\beta]_2$).

The haptoglobin used in the present embodiment may be a tetramer or higher molecule in which multiple dimers of $\alpha$ chains and $\beta$ chains are associated.

(Hemoglobin-Haptoglobin Complex)

When hemoglobin and haptoglobin form a complex, one molecule of tetrameric hemoglobin $[\alpha_2\beta_2]$ dissociates into two molecules of dimeric hemoglobin $[\alpha\beta]$, and each dimeric hemoglobin binds to haptoglobin. Human type 1-1 haptoglobin has two binding sites for dimeric hemoglobin in the molecule (one site for each $\beta$ chain). When human type 1-1 haptoglobin and tetrameric hemoglobin $[\alpha_2\beta_2]$ form a complex, therefore, it becomes a complex in which two units of dimeric hemoglobin $[\alpha\beta]$ are bound to one molecule of human type 1-1 haptoglobin.

Human type 2-1 haptoglobin and type 2-2 haptoglobin have two or more $\beta$ chains in the molecule, and can bind to dimeric hemoglobin $[\alpha\beta]$ depending on the number of $\beta$ chains.

Haptoglobin from horses, pigs, etc. has a structure like that of human type 1-1, and can form a complex in which two units of dimeric hemoglobin $[\alpha\beta]$ are bound to one molecule of haptoglobin.

In the present specification, the dissociation of one molecule of tetrameric hemoglobin $[\alpha_2\beta_2]$ into two molecules of dimeric hemoglobin $[\alpha\beta]$ when hemoglobin and haptoglobin form a complex is not included in the denaturation/decomposition of hemoglobin, which corresponds to a decrease in the storage stability of hemoglobin.

(N-linked Glycan)

An N-linked glycan refers to a glycan that binds to the amide nitrogen atom of a side chain of asparagine (Asn) in the consensus sequence of a protein (Asn-X-Ser/Thr: X is any amino acid except Pro).

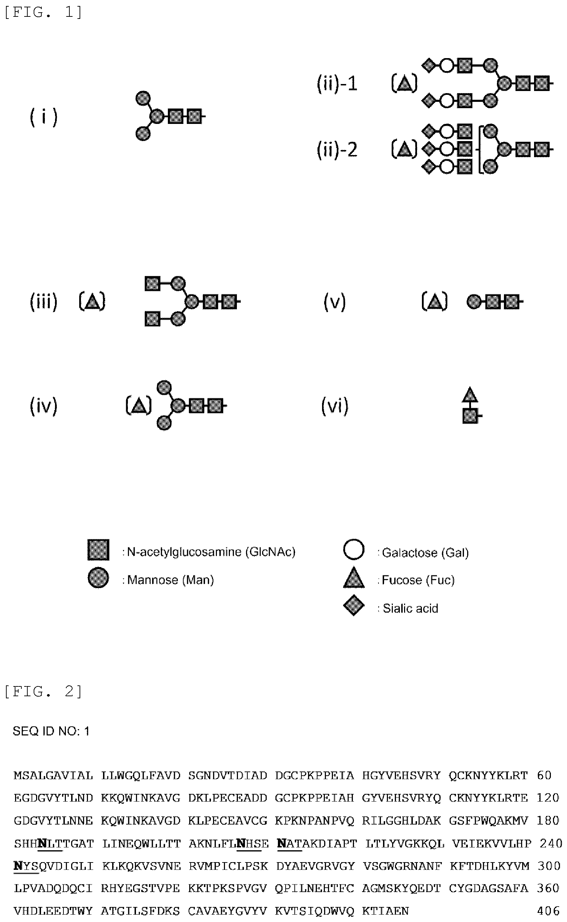

N-linked glycans usually have a basic skeleton called a core structure. As illustrated in FIG. 1(i), the core structure has a structure in which N-acetylglucosamine (GlcNAc) binds to the amide nitrogen atom of Asn, then binds to GlcNAc and mannose (Man), and two more mannose residues branch off and bind to this mannose, meaning that the structure is composed of two GlcNAc residues and three Man residues.

The aforementioned haptoglobin is known to be an N-linked glycoprotein including an N-linked glycan. Examples of N-linked glycans possessed by naturally-derived haptoglobin are illustrated in (ii)-1 and (ii)-2 of FIG. 1. The "naturally-derived haptoglobin" refers to haptoglobin that is originally expressed by the living organism without the aid of gene transfer or the like.

As illustrated in (ii)-1, the N-linked glycan possessed by naturally-derived haptoglobin has GlcNAc and galactose (Gal) added, in that order, to each of the Man termini of the two branched residues of the above core structure. In many cases, sialic acid is further added to the Gal.

One or more of the GlcNAcs contained in the N-linked glycan may be further modified with fucose (fucosylation). (ii)-1 illustrates an aspect in which the N-linked glycan has one fucose residue, but the GlcNAc to which it is bound is not specified. However, the GlcNAc that is fucosylated is typically the GlcNAc that is directly bound to Asn.

(ii)-2 of FIG. 1 illustrates an aspect in which a total of three GlcNAc-Gal-(sialic acid) glycans are bound to the Man termini of the two branched residues of the core structure and it is not specified which of the Man termini of the two residues are bound to two glycans. Other than this are the same as (ii)-1. Also known in a naturally-derived haptoglobin is an aspect in which a total of four GlcNAc-Gal-(sialic acid) glycans bound.

Table 1 lists preprohaptoglobin in each animal species and the positions on the asparagine to which the N-linked glycans are added. Note that each amino-acid sequence is available from UniProt (https://www.uniprot.org/), and all accession numbers indicated in the present specification are from UniProt.

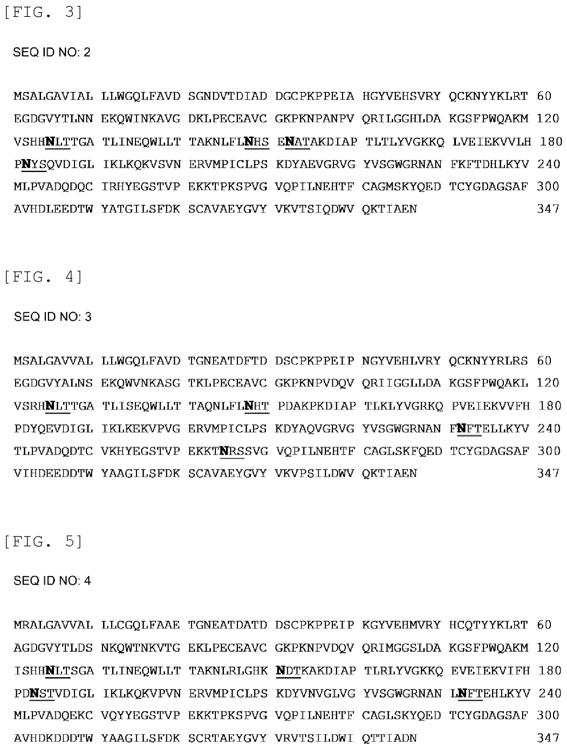

In humans, type 1 human haptoglobin, which contains a signal sequence-$\alpha$1 chain-$\beta$ chain, and type 2 human haptoglobin, which contains a signal sequence-$\alpha$2 chain-$\beta$ chain, are known, and the combination of their expression results in the above three serotypes (type 1-1, type 2-1, and type 2-2). Normally, in type 2 human haptoglobin having the amino-acid sequence of SEQ ID NO:1 (Accession No. P00738-1, SEQ ID NO:1, see FIG. 2), asparagine at each of positions 184, 207, 211, and 241 is glycosylated. In type 1 haptoglobin having the amino-acid sequence of SEQ ID NO:2, the amino acids at positions 38 to 96 of the above type 2 haptoglobin are deleted (Accession No. P00738-2, SEQ ID NO:2, see FIG. 3).

In equine haptoglobin having the amino-acid sequence of SEQ ID NO:3 (Accession No. F6XWM5, SEQ ID NO:3, see FIG. 4), asparagine at each of positions 125, 148, 232, and 265 is glycosylated.

In porcine haptoglobin having the amino-acid sequence of SEQ ID NO:4 (Accession No. Q8SPS7, SEQ ID NO:4, see FIG. 5), asparagine at each of positions 125, 151, 183, and 232 is glycosylated.

[Table 1]

| | Number of glycan chains | Positions on asparagine to which glycans are added | | | | UniProt Accession No. |
|---|---|---|---|---|---|---|
| Bovine | 2 | 286 | 316 | | | Q2TBU0 |
| Porcine | 4 | 125 | 151 | 183 | 232 | Q8SPS7 |
| Equine | 4 | 125 | 148 | 232 | 265 | F6XWM5 |
| Human type 1 | 4 | 125 | 148 | 152 | 182 | P00738-2 |
| Human type 2 | 4 | 184 | 207 | 211 | 241 | P00738-1 |

As demonstrated in the examples to be described later, hemoglobin forms a complex with haptoglobin to become a hemoglobin-haptoglobin complex, causing change in the measured values of hemoglobin concentration.

In this context, by using haptoglobin from which at least a portion of the N-linked glycan is deleted, it is possible to suppress change in the measured values of hemoglobin concentration, particularly a decrease in the value. Furthermore, the haptoglobin can suppress the denaturation/decomposition of hemoglobin, thereby stabilizing hemoglobin.

(Deglycosylated Haptoglobin)

In the haptoglobin according to one embodiment of the present invention, at least a portion of the N-linked glycan is deleted. Preferably, it has the storage stability of hemoglobin.

(Characteristic (1))

The haptoglobin according to the present embodiment has the following characteristic (1):

(1) at least a portion of the N-linked glycan is deleted.

In the present embodiment, "at least a portion of the N-linked glycan is deleted" means that at least a portion of the N-linked glycan is deleted compared to naturally-derived haptoglobin. As described previously, naturally-derived haptoglobin has an N-linked glycan added thereto.

The present inventors consider the mechanism of action by which the deletion of at least a portion of the glycan of haptoglobin suppresses change in the measured values of hemoglobin as follows.

Measurement of hemoglobin is often conducted using an immunological measurement method with an anti-hemoglobin antibody. For accurate measurement, the epitope of hemoglobin to which the antibody reacts has to be in a state of being exposed. When hemoglobin and haptoglobin form a complex, the N-linked glycan of haptoglobin may hide the epitope of hemoglobin, or may cause a physical obstacle to the binding of the antibody. In the haptoglobin from which the glycan of the N-linked glycan is deleted, therefore, it is presumed that such obstacles do not occur, and accurate measurement is possible.

However, the effect of the present embodiment is not limited to the above mechanism of action.

Whether or not a certain haptoglobin has the above characteristic (1) can be evaluated, for example, by treating the target haptoglobin with peptide-N-glycosidase (PNGase) and comparing the molecular weight of the β-chain between the treated haptoglobin and the untreated haptoglobin.

For example, it is known that the human type 1-1 haptoglobin ($[\alpha 1\beta]_2$) has an increased molecular weight of approximately 21,000 due to the N-linked glycan attached to its β-chain. This increase in molecular weight is approximately 10,500 per β-chain. Accordingly, as a guideline, when the difference in molecular weight per β-chain between PNGase-treated haptoglobin and untreated haptoglobin is 6,500 or less, the haptoglobin can be said to have the characteristic (1). In this case, the characteristic (1) can be preferably rephrased as follows:

(1a) the difference between the molecular weight of the β-chain in the haptoglobin treated with peptide-N-glycosidase (PNGase) and the molecular weight of the β-chain in then untreated haptoglobin is 6,500 or less.

When multiple peaks derived from β-chain haptoglobin are detected, the difference in molecular weight is compared between the peaks with the greatest signal intensity.

The above difference in molecular weight may be 6,500 or less (13,000 or less when converted to one molecule of human type 1-1 or equine $[\alpha\beta]_2$, or the like), but may also be 5,000 or less (10,000 or less), or even 2,500 (5,000 or less) or 1,550 (3,100 or less).

In the evaluation of whether or not the haptoglobin has the characteristic (1a), only the molecular weight of the haptoglobin β-chain is evaluated, so the haptoglobin may be denatured in the peptide-N-glycosidase treatment, unlike the method described later (method for producing haptoglobin). Measurement of the molecular weight may be performed, for example, by TOF MS analysis.

Regarding the above characteristic (1), "at least a portion of the N-linked glycan is deleted" may mean that for a certain N-linked glycan, a portion of that N-linked glycan is deleted, or that the entire N-linked glycan is deleted (i.e., that N-linked glycan is not present).

When a naturally-derived haptoglobin has multiple N-linked glycans, "at least a portion of the N-linked glycan is deleted" may mean that at least one of the N-linked glycans possessed by the naturally-derived haptoglobin is deleted in part or in whole. Furthermore, multiple N-linked glycans may be deleted in part or in whole, or all the N-linked glycans may be deleted in part or in whole. A haptoglobin from which all N-linked glycans are deleted means that the haptoglobin has no N-linked glycans at all.

On the other hand, examples of "haptoglobin from which a portion of the N-linked glycans is deleted" include haptoglobin from which a portion of an N-linked glycan is deleted, as illustrated in (iii) to (vi) of FIG. 1.

(iii) to (vi) of FIG. 1 illustrate those in which one fucose residue is added to the remaining N-linked glycan. However, depending on the number of GlcNAc residues in the remaining N-linked glycan, two or more fucose residues may be added, or no fucose may be added. Specific examples include:

(iii) one in which sialic acids and Gals are deleted and GlcNAcs are added to the core structure;
(iv) one in which sialic acids, Gals, and GlcNAc are deleted and only the core structure remains;
(v) one in which two branched Man residues in the core structure are deleted and two GlcNAc residues and one Man residue remain; and
(vi) one in which three Man residues and one GlcNAc residue in the core structure are deleted and one GlcNAc residue remains.

From the viewpoint of effectively exhibiting the effects of the present embodiment and from the viewpoint of easy preparation, the haptoglobin of the present embodiment is preferably one that does not have at least one of the multiple N-linked glycans; one that has no N-linked glycans at all; or one configured such that at least one or all of the multiple N-linked glycans is one N-acetylglucosamine residue or one in which one fucose residue is bound to one N-acetylglucosamine residue.

(Characteristic 2)

The haptoglobin of the present embodiment preferably further has the following characteristic (2):

(2) when 3.1 pmol of hemoglobin is stored at 37°C for 7 days in 500 μL of a buffer solution containing 3.1 pmol of the haptoglobin, the residual ratio of the hemoglobin is 70% or more.

In the present embodiment, the "residual ratio of hemoglobin (%)" can be calculated as follows:

(Hemoglobin concentration measured after storage at 37°C for 7 days/Hemoglobin concentration measured immediately after adding hemoglobin to the buffer solution) $\times$ 100.

Note that in the present specification, haptoglobin having the above characteristic (1) or characteristics (1) and (2) may be referred to as "deglycosylated haptoglobin" (which may be written as dHpt, hereinafter).

Haptoglobin having the above characteristic (2) can form a complex with hemoglobin to stabilize the hemoglobin. The residual ratio of hemoglobin in the characteristic (2) is preferably 75% or more and particularly preferably 80% or more.

Haptoglobin is one of the glycoproteins for which many examples of glycan analysis using various glycosidase treatments have been reported, but in such glycan analysis, it is common to denature glycoproteins containing haptoglobin before carrying out the glycosidase treatment. Therefore, haptoglobin subjected to the glycosidase treatment for glycan analysis does not usually satisfy the above characteristic (2).

In the present specification, unless otherwise stated, the amount of haptoglobin is expressed using the amount of substance when the molar equivalent to human hemoglobin is 1:1.

As described previously, human haptoglobin is known to be of type 1-1 ($[\alpha 1\beta]_2$), type 2-1 ($[\alpha 1\beta]_2[\alpha 2\beta]_n$, where n=0, 1, 2, ...), and type 2-2 ($[\alpha 2\beta]_m$, where m=3, 4, 5, ...), but in the present specification, the amount of haptoglobin that can form a complex, for example, with 1 μmol of human free hemoglobin is expressed as 1 μmol, regardless of whether it is type 1-1, type 2-1, or type 2-2.

By expressing the amount of haptoglobin using the amount of substance when the molar equivalent to human hemoglobin is 1:1, it is possible to specify the amount of haptoglobin regardless of its type, and it can also be quantified even when haptoglobin is contained in a mixture or the like.

The amount of haptoglobin can be quantified as the amount at which a predetermined amount of human free hemoglobin is completely bound to haptoglobin to form a complex and there is no more human free hemoglobin, and it can be measured using a measurement reagent specific to free hemoglobin. A specific measurement method will be described in the examples to be described later.

(Method for Producing Deglycosylated Haptoglobin)

Haptoglobin having the above characteristic (1) or characteristics (1) and (2) can be obtained, for example, using a method of treating haptoglobin having natural N-linked glycans with glycosidase to cleave and remove all or part of the N-linked glycans from the haptoglobin, resulting in haptoglobin from which at least a portion of the N-linked glycans is cleaved and removed by the glycosidase treatment. Additionally or alternatively, for example, a genetic engineering procedure may be used to modify the aforementioned consensus sequence specific to N-linked glycan addition contained in haptoglobin to obtain recombinant haptoglobin that is not subjected to N-linked glycan modification.

(Glycosidase Treatment)

In the present embodiment, "haptoglobin having a natural N-linked glycan" refers to haptoglobin having an N-linked glycan equivalent to the aforementioned naturally-derived haptoglobin. Examples of haptoglobin having a natural N-linked glycan for use include naturally-derived haptoglobin as well as recombinant haptoglobin in which haptoglobin is expressed in cultured cells (host cells).

Examples of naturally-derived haptoglobin for preferred use include haptoglobin derived from humans as well as from animals such as horses and pigs.

Examples of recombinant haptoglobin include recombinant haptoglobin obtained by co-expressing complement C1r subcomponent-like protein (C1r-LP) (see Schaer et al. BMC Biotechnology (2018) 18:15).

From the viewpoints of safety and cost, naturally-derived haptoglobin is preferred, and haptoglobin derived from humans, horses, or pigs is particularly preferred.

Here, when obtaining haptoglobin that does not have at least one of the multiple N-linked glycans (e.g., a haptoglobin having no N-linked glycans at all) among the haptoglobins having the characteristic (1), the glycosidase to be used is not particularly limited, but examples thereof include peptide-N-glycosidase (PNGase). Peptide-N-glycosidase is an enzyme that cleaves the glycosidic bond between a nitrogen atom and a sugar, and in N-linked glycans, it cleaves the bond between a GlcNAc residue and an asparagine residue. Examples of peptide-N-glycosidase include PNGase F.

When obtaining haptoglobin from which a portion of at least one N-linked glycans is deleted, the glycosidases that can be used and the N-linked glycan with a deleted portion obtained thereby are, although not particularly limited thereto, as exemplified below:

- sialidase and β-galactosidase: those in which sialic acids and Gals are deleted and GlcNAcs are added to the core structure ((iii) of FIG. 1);
- β-N-acetylglucosaminidase (e.g., β-(1-2,3,4,6)-N-glucosylaminidase) further: those in which sialic acids, Gals, and GlcNAc are deleted and only the core structure remains ((iv) of FIG. 1);
- α-mannosidase (e.g., α-(1-2,3,6)-mannosidase) further: those in which two branched Man residues in the core structure are deleted and two GlcNAc residues and one Man residue remain ((v) of FIG. 1); and
- endo-β-N-acetylglucosaminidase (ENGase): those in which three Man residues and one GlcNAc residue in the core structure are deleted and one GlcNAc residue remains.

As described previously, (iii) to (vi) of FIG. 1 illustrate those in which one fucose residue is added to the remaining N-linked glycan, but two or more fucose residues may be added, or no fucose may be added.

Among the above-described glycosidases, sialidase, β-galactosidase, β-(1-2,3,4,6)-N-glucosylaminidase, and α-(1-2,3,6)-mannosidase are known as exoglycosidases. When these are used, the desired N-linked glycans can be obtained by treating with glycosidases in the above-mentioned order. Provided that the above-mentioned order is followed, the glycosidases may be treated sequentially, or some or all the glycosidases may be treated simultaneously.

On the other hand, endo-β-N-acetylglucosaminidase (ENGase) is an endoglycosidase that cleaves the β1-4 bond between GlcNAcs, and in N-linked glycans, the above bond in the core structure is cleaved. Examples of the endo-β-N-acetylglucosaminidase include, but are not limited to, Endo S, Endo F3, Endo F2, Endo M, Endo Om, and Endo CC.

The aforementioned glycosidases are commercially available from New England Biolabs, Ludger, and others.

The deglycosylated haptoglobin of the present embodiment has, in addition to the characteristic (1), the characteristic (2) as a preferred aspect. In order to obtain haptoglobin having the characteristic (2), it is preferred not to denature the haptoglobin during the glycosidase treatment.

More specifically, the method preferably includes a step of subjecting haptoglobin having a natural N-linked glycan (preferably naturally-derived haptoglobin) to a glycosidase treatment, and the following (a) and (b) are satisfied:

(a) the naturally-derived haptoglobin is not subjected to a heat shock of 90°C or higher prior to the glycosidase treatment; and
(b) SDS is not allowed to coexist during the glycosidase treatment.

As described previously, in the glycan analysis of glycoproteins including haptoglobin, it is common to denature the glycoproteins including haptoglobin before carrying out the glycosidase treatment. The reason for this is believed to be that the denaturation of glycoproteins allows glycosidases to more readily access the glycans, thus increasing the reaction efficiency. However, when producing deglycosylated haptoglobin having the characteristic (2), it is preferred not to carry out the denaturing treatment, specifically the above treatments (a) and (b).

(Genetic Engineering Procedure)

Methods for deleting N-linked glycans using a genetic engineering procedure include a method in which a consensus sequence specific to N-linked glycan addition is modified to allow the host cell to express recombinant haptoglobin that does not undergo N-linked glycan modification.

The haptoglobin before modifying the consensus sequence may have an amino-acid sequence identical to any one of known haptoglobin amino-acid sequences, for example, SEQ ID NOs: 1 to 4, or have an amino-acid sequence that is 80% or more identical, more preferably 90% or more identical, and further preferably 95% or more identical.

Specific examples of the modification of the consensus sequence (Asn-X-Ser/Thr: X is any amino acid except Pro) include: substituting Asn or Ser/Thr in the consensus sequence with another amino acid; deleting any one or more amino acids in the consensus sequence; and inserting any one or more amino acids into the consensus sequence. The modification (substitution, deletion, or insertion) of the consensus sequence can be appropriately performed using a conventionally known genetic engineering procedure or a method similar thereto. When haptoglobin has multiple consensus sequences, it is sufficient to modify at least one consensus sequence, but it is preferred to modify two or more consensus sequences, and it is more preferred to modify all consensus sequences.

The modified haptoglobin gene obtained as described above can be expressed in the host cell and purified using a conventionally known method or a method similar thereto.

[Method and Solution for Stabilizing Hemoglobin]

The method for stabilizing hemoglobin according to one embodiment of the present invention includes a step of allowing hemoglobin to coexist with the above deglycosylated haptoglobin.

The solution for stabilizing hemoglobin according to one embodiment of the present invention includes the above deglycosylated haptoglobin.

Specifically, the haptoglobin (deglycosylated haptoglobin) used in the present embodiment has the following characteristic (1):

(1) at least a portion of an N-linked glycan is deleted.

The above characteristic (1) can be preferably rephrased as follows:
(1a) the difference between the molecular weight of the β-chain in the haptoglobin treated with peptide-N-glycosidase (PNGase) and the molecular weight of the β-chain in the untreated haptoglobin is 6,500 or less.

The haptoglobin used in the present embodiment preferably further has the following characteristic (2):
(2) when 3.1 pmol of hemoglobin is stored at 37°C for 7 days in 500 μL of a buffer solution containing 3.1 pmol of the haptoglobin, the residual ratio of the hemoglobin is 70% or more.

In the present specification, the "coexistence" of hemoglobin and deglycosylated haptoglobin includes a state in which hemoglobin and deglycosylated haptoglobin form a complex (hemoglobin-haptoglobin complex).

The method of allowing hemoglobin to coexist with deglycosylated haptoglobin is not particularly limited, but an example of the method is to disperse hemoglobin in a solution containing deglycosylated haptoglobin (i.e., a solution for stabilization). Here, the "dispersion" includes dissolution and suspension.

Examples of solutions for stabilizing hemoglobin include a preservation solution for preserving hemoglobin, a dilution solution for further diluting a sample in which hemoglobin is dispersed in a preservation solution, and a reaction solution in a kit or the like for detecting hemoglobin. The solution for stabilizing hemoglobin can also be used as a calibrator or control for use in the calibration or accuracy management of an automatic analyzer.

The hemoglobin to be stabilized may be that contained in a specimen derived from living organisms, such as blood, serum, plasma, urine, sputum, lymph, puncture fluid, spinal fluid, sweat, saliva, gastric fluid, lung lavage fluid, or feces.

The concentration of deglycosylated haptoglobin in the solution for stabilizing hemoglobin is not particularly limited, but can be, for example, 0.005 to 500 pmol/mL, 0.05 to 250 pmol/mL, or 0.5 to 50 pmol/mL.

The solution for stabilizing hemoglobin may be any of buffer solutions capable of maintaining a pH of 5 to 10, preferably 6 to 8, including Good's buffer solutions such as 2-morpholinoethanesulfonic acid (MES), hydroxyethylpiperazine-2-ethanesulfonic acid (HEPES), and piperazine-bis(2-ethanesulfonic acid) (PIPES), and it may also be a phosphate buffer solution, a Tris buffer solution, or a glycine buffer solution.

The solution for stabilizing hemoglobin according to the present embodiment may contain other components acceptable for stabilizing hemoglobin, in addition to the above deglycosylated haptoglobin. Examples of such components include additives such as antibacterial agents, pH adjusters, salts for adjusting ionic strength, surfactants, coagulation promoters, and publicly-known stabilizers used during protein storage. Antibacterial agents include sodium azide, antibiotics, and lytic enzymes. Additives include publicly-known substances known to have an action of stabilizing protein, such as amino acids such as histidine and lysine, albumin, protease inhibitors, and chelating agents such as ethylenediaminetetraacetic acid (EDTA).

(Immunological Measurement Method for Hemoglobin)

The immunological measurement method for hemoglobin according to one embodiment of the present invention includes:
a step of performing the immunological measurement using an anti-hemoglobin antibody under coexistence of the hemoglobin and the above deglycosylated haptoglobin.

The method for allowing the hemoglobin to coexist with the deglycosylated haptoglobin is not particularly limited, and for example, the methods described in the aforementioned method and solution for stabilization can be adopted.

Specifically, the haptoglobin (deglycosylated haptoglobin) used in the present embodiment has the following characteristic (1):

(1) at least a portion of an N-linked glycan is deleted.

The above characteristic (1) can be preferably rephrased as follows:
(1a) the difference between the molecular weight of the β-chain in the haptoglobin treated with peptide-N-glycosidase (PNGase) and the molecular weight of the β-chain in the untreated haptoglobin is 6,500 or less.

The haptoglobin used in the present embodiment preferably further has the following characteristic (2):
(2) when 3.1 pmol of hemoglobin is stored at 37°C for 7 days in 500 μL of a buffer solution containing 3.1 pmol of the haptoglobin, the residual ratio of the hemoglobin is 70% or more.

(Immunological Measurement Method)

The immunological measurement method for hemoglobin of the present embodiment is not particularly limited, provided that it is a method that utilizes an antigen-antibody reaction, that is, an immunological scheme, and examples thereof include immunoagglutination methods using immunonephelometry, immunoturbidimetry, etc. (e.g., latex agglutination method, gold colloid agglutination method, etc.), enzyme immunoassays (EIA methods) such as ELISA and bioluminescent enzyme immunoassay, radioimmunoassay (RIA), chemiluminescent immunoassay (CLIA), electrochemiluminescent immunoassay (ECLEIA), fluorescent immunoassay, immunochromatography, Western blot, and immunoblot, but are not limited to these. The measurement method of the present embodiment is preferably used for immunoagglutination methods, more preferably latex agglutination methods.

(Anti-hemoglobin Antibody)

The type of anti-hemoglobin antibody that can be used in the present embodiment is not particularly limited. For example, the animal species from which the antibody is derived is not particularly limited, and examples include antibodies derived from animals such as rabbits, goats, mice, rats, horses, and sheep. Any of polyclonal antibodies, monoclonal antibodies, or fragments thereof [e.g., F(ab')2, Fab, Fab', or Fv] may be used. These can be obtained by publicly-known methods, and for example, polyclonal antibodies can be obtained from the serum of an animal immunized with the object to be measured, and monoclonal antibodies can be obtained by cell fusion of the spleen or lymph node of an animal immunized with the object to be measured with myeloma cells.

(Insoluble Carrier)

At least one of the anti-hemoglobin antibodies used in the present embodiment may be supported on an insoluble carrier, and two or more of them may also be supported on an insoluble carrier.

Such an insoluble carrier is not particularly limited, provided that it can support the antibody, and can be appropriately selected depending on the type of the aforementioned immunological measurement method. For example, examples of insoluble carriers include plates and porous membranes that can be used in immunological schemes, as well as insoluble particles, and examples of insoluble particles include commonly used metal colloid particles such as colloidal gold particles, latex particles, silica particles, magnetic particles, fluorescent particles, and red blood cells. As insoluble particles, latex particles are preferred, and polystyrene-based latex particles are more preferred. The insoluble carrier is preferably particulate, and its average particle diameter is preferably 5 to 1,000 nm, more preferably 30 to 500 nm, and further preferably 75 to 350 nm, but it can be used without being limited to this range.

Supporting or carrying an antibody means that the antibody is immobilized by physical adsorption or chemical bond to the surface of the insoluble carrier. As a method of supporting (immobilization), for example, a publicly-known technique can be used to mix the antibody with the insoluble carrier particles and physically adsorb the antibody to the surface of the insoluble carrier particles, thereby immobilizing the antibody on the insoluble carrier particles. When using insoluble carrier particles to which amino or carboxyl groups are introduced onto the surface, the antibody can be immobilized on the surface of the insoluble carrier particles by chemical bonding using glutaraldehyde or a carboximide reagent.

The amount of antibody supported is not particularly limited, but may be 0.5 to 2,000 $\mu$g/mg latex or may also be 1 to 1,000 $\mu$g/mg latex or 2 to 500 $\mu$g/mg latex. The amount of antibody supported can be calculated by subtracting the amount of antibody after immobilization on the insoluble carrier from the amount of antibody before immobilization.

When two or more types of antihemoglobin antibodies are supported on the insoluble carrier, they may all be supported on the same insoluble carrier. A mixture of multiple insoluble carriers, each of which has one type of anti-hemoglobin antibody supported on one type of insoluble carrier, may also be used. In this case, the insoluble carriers used to support different types of antihemoglobin antibodies may be the same type of insoluble carrier, or may also be different types of insoluble carriers with different materials or particle diameters.

(Object to be Measured)

The object to be measured to which the immunological measurement method of the present embodiment is applied is not particularly limited, provided that it can contain hemoglobin. Such objects to be measured include: specimens derived from living organisms, such as blood, serum, plasma, urine, sputum, lymph, puncture fluid, spinal fluid, sweat, saliva, gastric fluid, lung lavage fluid, and feces; samples obtained by diluting or suspending these specimens; and the like.

Among these, feces, blood, serum, plasma, saliva, urine, and sputum are suitable as objects to be measured containing hemoglobin.

The haptoglobin of the present embodiment can suppress change in the measured values of hemoglobin caused by haptoglobin, particularly a decrease in the value, and furthermore, the haptoglobin can suppress the denaturation/-decomposition of hemoglobin, so it is preferred to measure hemoglobin in fecal specimens, where denaturation/decomposition of hemoglobin is particularly significant.

(Measurement)

The step of performing the immunological measurement can be appropriately adopted from publicly-known immunological measurements. The index to be measured can be appropriately set according to the immunological scheme adopted, and examples of indices to be measured include an amount of turbidity change over a predetermined time in the case of the immune agglutination method and an amount of color development/light absorption caused by the labeling of a labeled antibody in the case of the ELISA method. Thus, the method of measuring these can also adopt publicly-known methods, such as optical methodology, and a general-purpose optical measurement device can be used.

As an example, the immunological measurement of hemoglobin can be performed as follows. First, a specimen is added to a container containing a preservative solution to prepare a sample. The specimen may be stored in the container for any time, or the sample may be obtained by filtering the specimen-containing storage solution. Hemoglobin in the sample is then detected by an immunological measurement method, for example, latex agglutination. More specifically, a reagent containing latex particles supporting anti-hemoglobin antibodies on their surfaces is added to the sample. Before adding the reagent containing latex particles, the sample may be diluted with a diluent or a reaction solution may be added. In the present embodiment, the above-described stabilizing solution containing deglycosylated haptoglobin may be any of a storage solution, a diluent, and a reaction solution. It is particularly preferred that the storage solution should be the above-described stabilizing solution, and in this case, the diluent and/or reaction solution may or may not contain deglycosylated haptoglobin.

When hemoglobin is present in the sample, the anti-hemoglobin antibody recognizes the hemoglobin, and the latex particles supporting the antibody agglutinate. The change in turbidity due to agglutination is measured, and the hemoglobin concentration in the sample is obtained from a calibration curve created using a calibrator containing a known concentration of hemoglobin and/or hemoglobin-haptoglobin complex. The hemoglobin concentration in the sample can also be obtained from a calibration curve created based on the hemoglobin concentration of the calibrator.

The immunological measurement method according to the above embodiment can suppress change in the measurement values of hemoglobin, particularly false low values, due to haptoglobin by performing an antigen-antibody reaction for measuring hemoglobin using an anti-hemoglobin antibody under the coexistence with the above deglycosylated haptoglobin (corresponding to the method for suppressing change in measurement values of hemoglobin according to one embodiment of the present invention).

[Hemoglobin Measurement Kit]

The kit for immunologically measuring hemoglobin according to one embodiment of the present invention includes:

a hemoglobin stabilizing solution containing the above deglycosylated haptoglobin; and
a reagent containing an anti-hemoglobin antibody.

The aforementioned stabilizing solution can be used as the hemoglobin stabilizing solution used in the present embodiment.
Specifically, the haptoglobin used in the present embodiment has the following characteristic (1):

(1) at least a portion of an N-linked glycan is deleted.

The above characteristic (1) can be preferably rephrased as follows:
(1a) the difference between the molecular weight of the $\beta$-chain in the haptoglobin treated with peptide-N-glycosidase (PNGase) and the molecular weight of the $\beta$-chain in the untreated haptoglobin is 6,500 or less.
The haptoglobin used in the present embodiment preferably further has the following characteristic (2):
(2) when 3.1 pmol of hemoglobin is stored at 37°C for 7 days in 500 $\mu$L of a buffer solution containing 3.1 pmol of the haptoglobin, the residual ratio of the hemoglobin is 70% or more.

(Reagent)

The reagent used in the present embodiment is not particularly limited, provided that it contains an anti-hemoglobin antibody and can immunologically measure hemoglobin. It can be a reagent that uses a method that utilizes the aforementioned antigen-antibody reaction, such as an immunoagglutination method (e.g., latex agglutination method, gold colloid agglutination method, etc.), an ELISA method, an immunochromatography method, etc. Among these, a reagent for an immunoagglutination method is preferred, and a reagent for a latex agglutination method is more preferred.
The anti-hemoglobin antibody used in the present embodiment can be the antibody described in the aforementioned immunological measurement method.
At least one of the anti-hemoglobin antibodies used in the present embodiment may be supported on an insoluble carrier, and two or more of them may also be supported on an insoluble carrier. Such an insoluble carrier may be an insoluble particle. The types of insoluble carriers and insoluble particles, the method of supporting the antibody, etc. are as described in the aforementioned immunological measurement method.

(Example of Reagent Configuration)

The measurement reagent of the present embodiment may be configured, for example, as a two-reagent system of a reagent (first reagent) that does not contain an insoluble carrier and a reagent (second reagent) that contains an insoluble carrier supporting an antibody (antibody-supporting insoluble carrier), or as a single-reagent system of only a reagent that contains an antibody-supporting insoluble carrier.

Here, the first reagent can be used to adjust the measurement environment, such as by using it as a diluent to adjust the concentration of the object to be measured or foreign substances in the reaction system or to adjust the reaction rate, etc. The second reagent contains an antibody-supporting insoluble carrier, and is mixed with the first reagent and the sample to cause an immune agglutination reaction. The first and second reagents can contain pH buffers, salts, surfactants, agglutination enhancer, preservatives, etc. as appropriate. The pH during the agglutination reaction is preferably 5 to 9.

The measurement reagent is mixed with the sample to obtain a reaction solution, and the concentration of the insoluble carrier in the reaction solution can be appropriately selected, for example, from a range of 0.0001 mg/mL to 10 mg/mL, depending on the particle diameter of the insoluble carrier used and the design of the entire measurement system. The concentration of the insoluble carrier supporting the anti-hemoglobin antibody in the measurement reagent may be 0.01 to 5 mg/mL or 0.05 to 1 mg/mL. The concentration of the insoluble carrier in the second reagent can be appropriately selected according to the dilution ratio because it is diluted by mixing with the first reagent, the sample, etc. when used. For example, when used after 2-fold dilution, it can be appropriately adjusted to 0.0002 to 20 mg/mL, and when used after 3-fold dilution, it can be appropriately adjusted to 0.0003 to 30 mg/mL.

(Other Configurations)

The hemoglobin measurement kit according to the present embodiment may include, in addition to the above stabilizing solution and measurement reagent, configurations such as a calibrator and a control, and may also include configurations such as an instrument and a container for collecting a specimen. Furthermore, it may include an attached document such as an instruction manual for use and/or an instruction manual that describes the range of reference values (reference range) that serve as a reference for determining whether hemoglobin is positive or negative (e.g., in the form of being printed on paper, plastic, or the like and included in the kit, or in the form of being viewed or downloaded via the Internet, etc.).

It should be appreciated that the aforementioned embodiments are described to facilitate understanding of the present invention and are not described to limit the present invention. It is therefore intended that the elements disclosed in the above embodiments include all design changes and equivalents to fall within the technical scope of the present invention.

(Reference Examples)

The free hemoglobin measurement reagent used in the examples to be described later was obtained according to the following reference examples.

Reference Example 1: Preparation of anti-human hemoglobin monoclonal antibody

(1) Immunization of mice

Mice were immunized with hemoglobin. After each immunization, the antibody titer of the mice was measured by a two-antibody RIA method using hemoglobin labeled with [125]I. As a result, mice with high antiserum titers were selected.

(2) Cell fusion

The spleens were removed from the selected mice, and splenocytes were prepared. The prepared splenocytes and mouse myeloma cells were fused by an electrofusion method, suspended in a fusion cell selection medium, and seeded on a 96-well microplate.

(3) Screening of monoclonal antibody-producing cell lines

Ten days after cell fusion, anti-human hemoglobin monoclonal antibody-producing cells were screened by a two-antibody RIA method using $^{125}$I-labeled hemoglobin, and 10 clones were obtained.

Reference Example 2: Confirmation of specificity of anti-hemoglobin antibodies

Human hemoglobin (available from EIKEN CHEMICAL CO., LTD.) purified from human type O blood was electrophoresed using a polyacrylamide precast gel for electrophoresis (available from ATTO CO., LTD.) and an AE-6530 MiniSlab Electrophoresis Tank (available from ATTO CO., LTD.). The electrophoresed proteins were transferred to a PVDF membrane using a TransBlot SD Cell (available from Bio-Rad Laboratories, Inc.).

Western blotting was performed using the antibody (1 $\mu$g/mL) obtained in Reference Example 1 as the primary antibody and an anti-mouse IgG antibody recognizing antibody labeled with HRP (rabbit-derived polyclonal antibody, available from Cappel) as the secondary antibody, and band images were acquired by emitting HRP using Western Lightning ECL Pro (available from PerkinElmer). The band images were analyzed using CS Analyzer ver. 3.0 (available from ATTO CO., LTD.), and the band luminance of the electrophoretic lane was calculated as an integrated value using the measurement mode: designated area zone densitometry.

Next, the integrated band luminance values of the $\alpha$-chain and $\beta$-chain were calculated from the band luminance of the Western blotting. To correct the band luminance, the $\alpha$-chain band integrated value was multiplied by a coefficient of 0.783 calculated by the method to be described below.

The band luminance ratio of each subunit was obtained when the $\alpha$-chain integrated value + $\beta$-chain integrated value was set to 100%. From these results, antibodies that showed an $\alpha$-chain band integrated value ratio of 75% or more were determined to be hemoglobin $\alpha$-chain specific antibodies, and antibodies that showed a $\beta$-chain band integrated value ratio of 75% or more were determined to be hemoglobin $\beta$-chain specific antibodies. The results are listed in Table 2.

To correct the band luminance, human hemoglobin was electrophoresed in the same manner as above using a polyacrylamide precast gel for electrophoresis (available from ATTO CO., LTD.) and an AE-6530 MiniSlab Electrophoresis Tank (available from ATTO CO., LTD.), and CBB staining was performed. The integrated values of the band luminance of the $\alpha$-chain and $\beta$-chain in the obtained electrophoretic image were calculated, and the band luminance ratio of each subunit was calculated when the $\alpha$-chain integrated value + $\beta$-chain integrated value was set to 100%. From these results, the band luminance of the $\beta$-chain and $\alpha$-chain was 43.9%:56.1%, and to normalize the band luminance, the $\alpha$-chain band integrated value was multiplied by a coefficient of 0.783.

[Table 2]

| Sample | Name of antibody | Recognized site | Integrated value | | Ratio | |
|---|---|---|---|---|---|---|
| | | | $\beta$ | $\alpha$ (after correction) | $\beta$ | $\alpha$ |
| 1 | No.4 | $\beta$ | 18732 | 438 | 97.7% | 2.3% |
| 2 | No.11 | $\beta$ | 160859 | 0 | 100.0% | 0.0% |
| 3 | No.16 | $\alpha$ | 434 | 3180 | 12.0% | 88.0% |
| 4 | No.8 | $\alpha$ | 465 | 2913 | 13.8% | 86.2% |
| 5 | No.1 | $\alpha$ | 992 | 9466 | 9.5% | 90.5% |
| 6 | No.12 | $\alpha$ | 312 | 1038 | 23.1% | 76.9% |
| 7 | No.21 | $\beta$ | 24696 | 362 | 98.6% | 1.4% |
| 8 | No.23 | $\beta$ | 9750 | 352 | 96.5% | 3.5% |
| 9 | No.24 | $\beta$ | 1118 | 122 | 90.2% | 9.8% |
| 10 | No.32 | $\beta$ | 6111 | 197 | 96.9% | 3.1% |

As listed in Table 2, among the antibodies obtained in Reference Example 1, Nos. 1, 8, 12, and 16 were recognized as $\alpha$-chain specific antibodies because the ratio of the $\alpha$-chain integrated value to the total of the $\alpha$-chain integrated value and the $\beta$-chain integrated value was 75% or more.

In contrast, Nos. 4, 11, 21, 23, 24, and 32 were recognized as $\beta$-chain specific antibodies because the ratio of the $\beta$-chain integrated value to the total of the $\alpha$-chain integrated value and the $\beta$-chain integrated value was 75% or more.

Reference Example 3: Reactivity of monoclonal antibodies

Using the antibodies obtained in Reference Example 1, the reactivity to free hemoglobin and the reactivity to hemoglobin-haptoglobin complexes were evaluated. Using the method described below, each anti-human hemoglobin monoclonal antibody was immobilized on polystyrene latex particles, and the degree of agglutination when reacted with a sample containing hemoglobin or a hemoglobin-haptoglobin complex was compared.

(1) Immobilization of monoclonal antibodies on polystyrene latex particles

Immobilization of antibodies on polystyrene latex particles was carried out using a publicly-known technique. Each anti-hemoglobin monoclonal antibody was mixed with polystyrene latex particles (particle diameter 200 nm) and immobilized, and an antibody-supporting polystyrene latex particle solution was prepared by supporting the anti-hemoglobin monoclonal antibody on the surface of the polystyrene latex particles.

(2) Preparation of sample

The sample of hemoglobin-haptoglobin complex was prepared by mixing equal volumes of 32.3 pmol/mL hemoglobin and 32.3 pmol/mL haptoglobin in 50 mM HEPES buffer solution (pH 7.4). The hemoglobin-only sample was prepared and used by mixing 16.1 pmol/mL hemoglobin in 50 mM HEPES buffer (pH 7.4).

(3) Measurement method for latex agglutination

The agglutination reaction was performed using the wells of a 96-well flat-bottom microplate. Specifically, 100 μL of 50 mM HEPES buffer solution (pH 7.4) was dispensed into each well of the microplate, 50 μL of polystyrene latex particle solution with each antibody immobilized thereon was added, and then 30 μL of the sample prepared in (2) was added. Using an absorbance microplate reader (Tecan Japan), the absorbance was measured at a wavelength of 660 nm 10 seconds and 5 minutes and 10 seconds after the addition of the sample, and the difference was used as an index of agglutination. Furthermore, the ratio of the agglutination reactivity to the hemoglobin to the agglutination reactivity to the hemoglobin-haptoglobin complex was calculated.
The results are listed in Table 3.

[Table 3]

| Combination of monoclonal antibodies | | Hemoglobin ($\varDelta$OD×10,000) | Hemoglobin -haptoglobin complex ($\varDelta$OD×10,000) | Ratio |
|---|---|---|---|---|
| No.1 | No.32 | 1790 | 30 | 1.68% |
| No.8 | No.4 | 1380 | 50 | 3.62% |
| No.8 | No.21 | 1080 | 0 | 0.00% |
| No.8 | No.23 | 540 | 0 | 0.00% |
| No.8 | No.24 | 680 | 0 | 0.00% |
| No.8 | No.32 | 580 | 0 | 0.00% |
| No.16 | No.4 | 2680 | 20 | 0.75% |
| No.16 | No.11 | 3120 | 80 | 2.56% |
| No.16 | No.23 | 1280 | 0 | 0.00% |
| No.16 | No.24 | 1700 | 0 | 0.00% |
| No.16 | No.32 | 1950 | 0 | 0.00% |

As listed in Table 3, a specific reaction was recognized with free hemoglobin in the combination of hemoglobin α-chain specific antibody and hemoglobin β-chain specific antibody. The reactivity with the hemoglobin-haptoglobin complex was 15% or less of the reactivity with free hemoglobin.

Reference Example 4: Free-hemoglobin measurement reagent

An immunoagglutination assay reagent was prepared using the antibody obtained in Reference Example 1.
As measurement reagents, the first and second reagents were prepared. As the first reagent, 50 mM HEPES buffer solution (pH 7.4) was used. As the second reagent, polystyrene latexes each supporting the anti-hemoglobin monoclonal antibody (anti-Hb antibody) of Reference Example 2 were mixed to adjust the latex concentration to 1.0 mg/mL to prepare an immunoagglutination assay reagent.

An antibody-supporting insoluble carrier was prepared by mixing each anti-hemoglobin monoclonal antibody with polystyrene latex particles (average particle diameter 100 nm) to support the anti-hemoglobin monoclonal antibody on the surface of the polystyrene latex particles. In this reagent, No. 16 was used as the $\alpha$-chain specific anti-hemoglobin monoclonal antibody, and No. 11 was used as the $\beta$-chain specific anti-hemoglobin monoclonal antibody.

Reference Example 5: Comparison with other measurement methods

A comparison of measurement methods was conducted using a specimen containing a mixture of free hemoglobin and hemoglobin-haptoglobin complex.

(1) Preparation of purified free hemoglobin and hemoglobin-haptoglobin complex

Total human hemoglobin routine reference standard JCCRM912-3H (available from Reference Material Institute for Clinical Chemistry Standards) was diluted to 2.0 mg/mL with physiological saline.

In addition, 1 mg of freeze-dried human pooled plasma-derived haptoglobin (available from SIGMA-ALDRICH) was dissolved in 303 $\mu$L of physiological saline to prepare a haptoglobin solution (3.3 mg/mL).

(2) Mixing of free hemoglobin and hemoglobin-haptoglobin complex

A fixed amount of free hemoglobin (free-Hb) adjusted to 2.0 mg/mL was mixed with haptoglobin (Hpt) solution adjusted to 3.3 mg/mL in the volumes listed in Table 4 to prepare solutions (specimens) with different mixing ratios. In Table 4, "free-Complex" represents the molar ratio of free hemoglobin to hemoglobin-haptoglobin complex.

[Table 4]

| free-Complex | 0 : 1 | 1 : 3 | 1 : 1 | 3 : 1 | 1 : 0 |
|---|---|---|---|---|---|
| free-Hb (2.0 mg/mL) | 200 $\mu$L | 200 $\mu$L | 200 $\mu$L | 200 $\mu$L | 200 $\mu$L |
| Hpt (3.3 mg/mL) | 200 $\mu$L | 150 $\mu$L | 100 $\mu$L | 50 $\mu$L | 0 $\mu$L |
| Physiological saline | 0 $\mu$L | 50 $\mu$L | 100 $\mu$L | 150 $\mu$L | 200 $\mu$L |
| Total | 400 $\mu$L | 400 $\mu$L | 400 $\mu$L | 400 $\mu$L | 400 $\mu$L |
| free-Hb concentration (mg/mL) | 0.00 | 0.25 | 0.50 | 0.75 | 1.00 |

(3) Measurement of free hemoglobin/hemoglobin-haptoglobin complex mixture liquid

A commercially available hemoglobin colorimetric assay reagent was used and compared with the immunoagglutination assay reagent prepared in Reference Example 4.

The hemoglobin colorimetric assay reagent is a reagent that dissolves red blood cell membranes with sodium lauryl sulfate and quantifies the eluted hemoglobin by measuring its absorbance. However, the hemolysis step was omitted in this test. Each mixture liquid (specimen) of free hemoglobin (free-Hb)/hemoglobin-haptoglobin (Hb-Hp) complex prepared in the above (2) was mixed with the hemoglobin colorimetric assay reagent and measured at 540 nm using a spectrophotometer (UV-1900 available from Shimadzu Corporation).

On the other hand, regarding the immunoagglutination assay reagent (Latex reagent) prepared in Reference Example 4, each mixture liquid (specimen) prepared in the above (2) was diluted 50-fold with physiological saline, and the measurement was performed using an automatic biochemical analyzer JCA-BM6070 under the following conditions: Specimen volume: 1.0 $\mu$L, First reagent: 50 $\mu$L, Second reagent: 50 $\mu$L, and Measurement wavelength: 658 nm

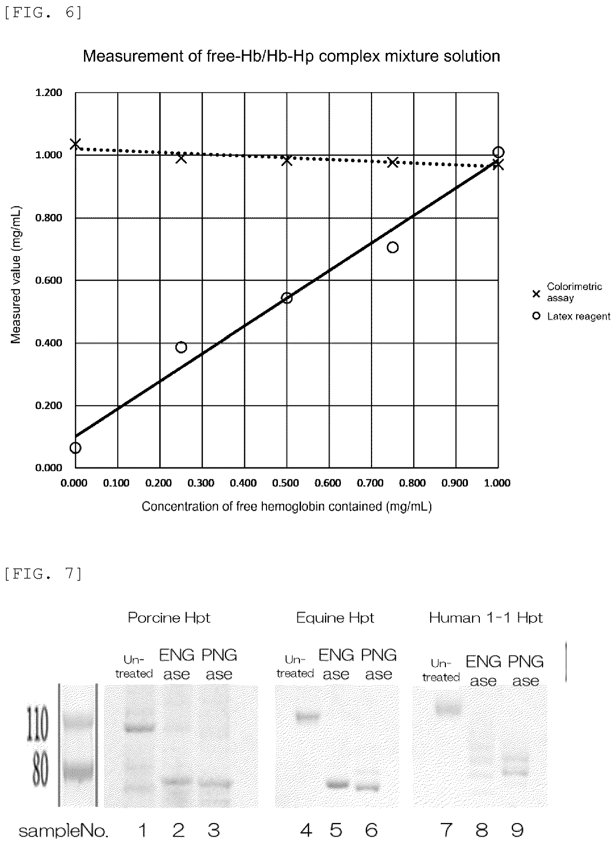

The results are listed in Table 5 and FIG. 6.

[Table 5]

| free-Hb concentration (mg/mL) | 0.000 | 0.250 | 0.500 | 0.750 | 1.000 |
|---|---|---|---|---|---|
| Colorimetric assay | 1.036 | 0.990 | 0.984 | 0.977 | 0.971 |
| Latex reagent | 0.065 | 0.387 | 0.544 | 0.706 | 1.010 |

Commercially available hemoglobin colorimetric quantitative reagents did not change the measured value even when the ratio of free hemoglobin to hemoglobin-haptoglobin complexes changed, and it was not possible to distinguish and measure the two. In contrast, the immunoagglutination assay reagent (latex reagent) of Reference Example 4 was not affected by the amount of hemoglobin-haptoglobin complex, and a measured value proportional to the free hemoglobin concentration was obtained.

The above results have demonstrated that the latex reagent of Reference Example 4 specifically measures free hemoglobin. This has demonstrated that free hemoglobin can be specifically measured by using a combination of hemoglobin $\alpha$-chain specific antibody and hemoglobin $\beta$-chain specific antibody.

**Examples**

The present invention will be described in more detail below by illustrating preparation examples, testing examples, etc., but the present invention is not limited to the following preparation examples, testing examples, etc.

Preparation Example 1: Preparation of deglycosylated haptoglobin (dHpt)

(1) Natural haptoglobin

Human Hpt (type 1-1), equine Hpt, and porcine Hpt purified from human serum, equine serum, and porcine serum, respectively, were used as naturally-derived haptoglobin (Hpt).

(2) ENGase treatment

To 20 $\mu$g of each type of Hpt, 64 U of Endo F2 (Endo-$\beta$-N-acetylglucosaminidase F2 available from New England Biolabs) was added, and the mixture was treated at 37°C for 5 days in 40 $\mu$L of GlycoBufer 1 (5 mM CaCl$_2$/50 mM sodium acetate, pH 5.5) (available from New England Biolabs) to obtain ENGase-treated Hpt (dHpt).

(3) PNGase treatment

To 20 $\mu$g of each type of Hpt, 4000U of PNGase F (Peptide-N-Glycosidase F available from New England Biolabs) was added, and the mixture was treated at 37°C for 5 days in 40 $\mu$L of GlycoBufer 2 (50mM sodium phosphate, pH7.5) (available from New England Biolabs) to obtain PNGase-treated Hpt (dHpt).

Testing Example 1: Confirmation of deglycosylation (SDS-PAGE)

The samples used were various types of the ENGase-treated and PNGase-treated Hpt (dHpt) obtained in Preparation Example 1 and various types of the glycosidase-untreated Hpt as controls.

The electrophoresis sample was obtained by mixing 8 $\mu$L of 200 $\mu$g/mL Hpt solution with 2 $\mu$L of 5×SDS-PAGE Sample Buffer (5% SDS, 25% glycerol, 0.05% bromophenol blue, 0.5M Tris/HCl, pH 6.8).

To a polyacrylamide gel (e-PAGEL HR 7.5% available from ATTO CO., LTD.), 10 $\mu$L of the electrophoresis sample obtained was applied. Electrophoresis was performed for 60 minutes at a constant current of 60 mA using a Mini-Slab Electrophoresis Tank and a Power Station Ghibli I (both available from ATTO CO., LTD.). The electrophoresis buffer used was Tris-Glycine-SDS Buffer powder (available from Takara Bio Inc.) dissolved as specified.

After electrophoresis, staining is carried out with TaKaRa CBB Protein Safe Stain (available from Takara Bio Inc.) for 30 minutes and then destained with purified water.

The results are illustrated in FIG. 7.

As illustrated in FIG. 7, in all cases of porcine Hpt, equine Hpt, and human Hpt, the molecular weight of the Hpt treated with ENGase or PNGase was reduced compared to the glycosidase-untreated samples, confirming the deglycosylation.

Testing Example 2: Confirmation of deglycosylation (MALDI-TOF MS)

The samples used were equine Hpt treated with ENGase and PNGase (dHpt) obtained in Preparation Example 1 and equine Hpt untreated with glycosidase as a control.

A mixture of 1 $\mu$L of 200 $\mu$g/mL equine Hpt with an equal volume of matrix (2',4'-dihydroxyacetophenone (DHAP) saturated with 100% acetonitrile) was used, and 1 $\mu$L of the mixture was dropped onto a Big Anchor plate. After drying the sample, it was analyzed by MALDI-TOF MS (instrument used: Ultraflextreme available from Bruker) (measurement conditions: linear positive mode).

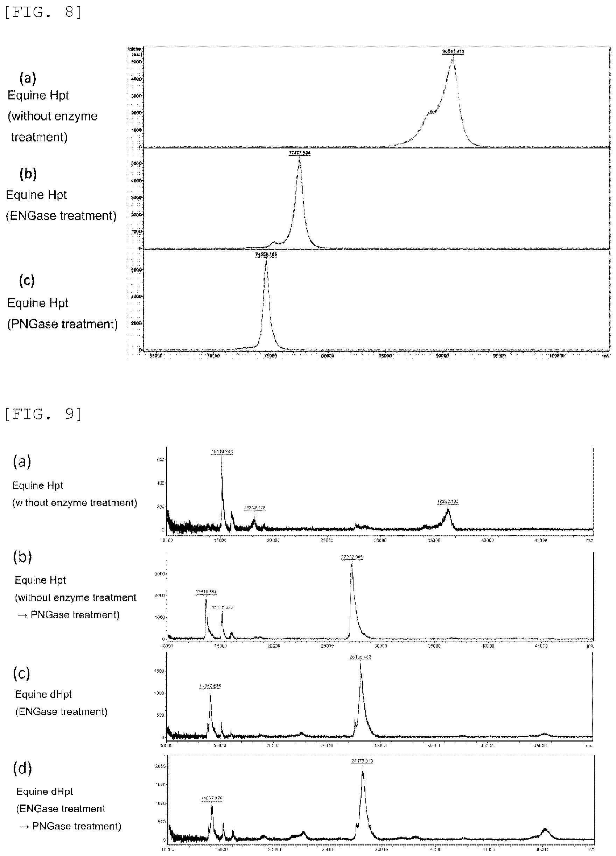

The results are illustrated in FIG. 8.

As illustrated in FIG. 8, the molecular weight of the Hpt treated with ENGase or PNGase was reduced compared to the glycosidase-untreated sample, confirming the deglycosylation.

The equine Hpt used in this test has a $[\alpha\beta]_2$ structure, that is, it has two β chains in one molecule.

Testing Example 3: Confirmation of deglycosylation (MALDI-TOF MS, reduced PNGase treatment)

The samples used were ENGase-treated equine Hpt (dHpt) obtained in Preparation Example 1 and equine Hpt untreated with glycosidase as controls.

Each 25 μg of equine Hpt was reduced by heating at 100°C for 3 minutes in 5 μL of Denaturation Buffer (0.5% SDS, 0.5 mol/L Tris-HCl (pH 8.6), 0.75% 2-mercaptoethanol (added when used), available from Takara Bio Inc.). After adding NP-40 to a final concentration of 1%, 1 mU of PNGase F (Peptide-N-Glycosidase F available from Takara Bio Inc.) was added to the reduced sample, and the total volume was adjusted to 25 μL with purified water, followed by treatment at 37°C for 24 hours.

For equine dHpt and glycosidase-untreated equine Hpt, samples that were reduced and then treated with PNGase, and samples that were reduced but not treated with PNGase, were prepared. The obtained samples were analyzed by MALDI-TOF MS as in Testing Example 2. Since haptoglobin is reduced in this test, the α chain and β chain are detected as separate molecules.

The results are illustrated in FIG. 9.

As illustrated in FIG. 9, (a) the molecular weight of the β-chain in glycosidase-untreated equine Hpt was approximately 36,000, whereas (b) the molecular weight of the β-chain was reduced to approximately 27,000 by treating it with PNGase. On the other hand, (c) equine dHpt that had been deglycosylated with ENGase and (d) equine dHpt that had been further treated with PNGase showed almost no difference in the molecular weight of the β-chain. In other words, it has been confirmed that the difference in molecular weight of the β-chain of deglycosylated haptoglobin with or without further PNGase treatment is 6,500 or less.

Thus, it has been shown that it is possible to evaluate whether haptoglobin is deglycosylated by comparing the molecular weight of the haptoglobin β-chain with or without PNGase treatment.

Testing Example 4: Quantification of haptoglobin using free hemoglobin reagent

(1) Sample preparation

A 10.5 mg/mL purified hemoglobin (Hb) solution purified from human blood was diluted with diluent (0.1% BSA/0.9% NaCl/0.1% NaN$_3$/50 mM HEPES, pH 7.0) to prepare an approximately 120 μg/mL Hb solution.

Original solutions of purified equine Hpt, purified human Hpt, and purified porcine Hpt, as well as an original solution of ENGase-treated equine Hpt (equine dHpt), were diluted with diluent to 2% (vol/vol) to prepare various Hpt solutions.

A sample was prepared by mixing 200 μL of the above Hb solution and 200 μL of the above Hpt solution. The obtained sample contains 59.4 μg/mL (=921 pmol/mL) of Hb and 1% (vol/vol) of Hpt of the original solution. The amount of Hb in the sample was determined by measuring a sample prepared by mixing 200 μL of the above Hb solution with 200 μL of dilution solution using the method described below.

Furthermore, a series of samples with different Hpt concentrations were prepared in the same manner as above, except that the Hpt concentrations in the samples were as listed in Table 6A. As a control, a sample was prepared to which dilution solution was added without adding Hpt.

(2) Measurement

The amount of free-Hb in these samples was measured using the free hemoglobin measurement reagent of Reference Example 4. Measurements were conducted using an automatic biochemical analyzer JCA-BM6070/C (BioMajesty 6070 G) (available from JEOL Ltd.) under the following conditions:

Analytical sample volume: 5.0 μL, First reagent: 50 μL, Second reagent: 50 μL, Measurement wavelength: 658 nm, Dilution ratio: 5-fold
*Analytical sample: Obtained by diluting the sample with physiological saline at the specified dilution ratio.

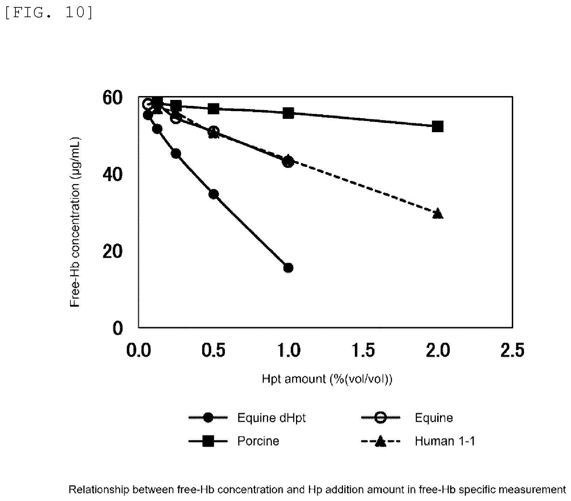

The measurement results are illustrated and listed in FIG. 10 and Table 6A. From the measurement results, an approximate straight line expressed by the following formula was obtained.

$$Y=aX+b$$

Y: Free-Hb concentration ($\mu$g/mL), a: Slope, X: Hpt concentration (% (vol/vol)), b: Intercept

Then, the amount of Hpt original solution added to the sample when the free-Hb concentration became 0 $\mu$g/mL was calculated. The Hpt concentration in the Hpt original solution was then calculated (Table 6B).

Here, the amount of Hpt that makes the free-Hb concentration 0 $\mu$g/mL is the amount of Hpt at which all Hb in the sample binds to Hpt to form Hb-Hpt complexes and there is no free-Hb. As such, even if Hpt is contained in a mixture or the like, it is possible to quantitate Hpt as a molar equivalent to human Hb.

The results are illustrated and listed in FIG. 10 and Tables 6A and 6B.

[Table 6A]

| Type of Hpt added | | Amount of Hpt added (% (vol/vol) ) | | | | | |
|---|---|---|---|---|---|---|---|
| | | 2.000 | 1.000 | 0.500 | 0.250 | 0.125 | 0.063 |
| Equine dHpt | free-Hb concentration ($\mu$g/mL) | | 15.6 | 34.7 | 45.3 | 51.8 | 55.4 |
| Equine | | | 43.2 | 51.0 | 54.6 | 58.3 | 58.2 |
| Porcine | | 52.4 | 55.9 | 57.0 | 57.8 | 58.6 | |
| Human | | 29.8 | 43.7 | 50.8 | 55.9 | 57.1 | |

[Table 6B]

| Type of Hpt added | Amount of Hpt original solution when free-Hb concentration became 0% (vol/vol) | Hpt concentration in Hpt original solution (nmol/mL) |
|---|---|---|
| Equine dHpt | 1.35 | 68 |
| Equine | 3.62 | 25 |
| Porcine | 18.65 | 5 |
| Human | 4.01 | 23 |

As illustrated in FIG. 10 and listed in Tables 6A and 6B, equine dHpt retained the ability to bind to free-Hb, like various non-deglycosylated Hpts, and the Hpt concentration was able to be quantified as a molar equivalent to human Hb.

Testing Example 5: Hb stability test (without stool)

(1) Sample preparation

An 80 $\mu$g/mL purified human Hb solution was diluted with dilution solution (0.1% BSA/0.9% NaCl/0.1% NaN$_3$/50mM HEPES, pH 7.0) to prepare an 800 ng/mL (12.4 pmol/mL) Hb solution.

Original solutions of purified porcine Hpt (without deglycosylation), ENGase-treated porcine Hpt, and PNGase-treated porcine Hpt were diluted with the above dilution solution to prepare Hpt solutions. The Hpt concentrations (molar equivalents to human Hb) of these Hpts were all calculated according to the above method of Testing Example 4, and the concentrations were adjusted based on this.

A sample was prepared by mixing 250 $\mu$L of the above Hb solution and 250 $\mu$L of the above Hpt solution. The obtained sample is 500 $\mu$L and contains 200 ng (3.1 pmol) of Hb and 3.1 pmol of each type of Hpt.

In the above sample, 3.1 pmol of Hpt is a necessary and sufficient amount to convert all 200 ng (3.1 pmol) of Hb in the sample into Hb-Hpt complex. This amount of Hpt (3.1 pmol) was defined as 1 Unit in this test, and samples were prepared with 1.0, 0.5, 0.25, and 0.125 Units of Hpt added in this test.

Samples of equine Hpt were prepared in the same manner as for the various porcine Hpts.

Samples of human Hpt were prepared in the same manner as for the various porcine Hpts, and samples with 2 Units of Hpt added were also prepared.

Furthermore, samples with no Hpt added but with dilution solution added (no Hpt added) were also prepared as controls.

(2) Measurement

The Hb values of these samples were measured using an Hb measurement reagent (OC-Hemodia(R) Auto III 'Eiken' available from EIKEN CHEMICAL CO., LTD.) and a fully automated fecal occult blood immunochemical analyzer (OC Sensor DIANA available from EIKEN CHEMICAL CO., LTD.). The measured values were used as the initial starting values.

The samples with the initial starting values measured were then stored at 37°C for 7 and 14 days, and the Hb values were measured again.

The initial starting values were compared to the initial starting values of samples with no Hpt added, and the "ratio to no Hpt added" was obtained to evaluate the change in Hb measurement value due to the addition of Hpt.

Ratio to no Hpt added (%) = (Hemoglobin concentration of sample with each amount of haptoglobin added/ Concentration of sample with no haptoglobin added) $\times$ 100

In addition, the measurement values after 7 and 14 days of storage were compared to respective initial starting values, and the "residual ratio of hemoglobin" was calculated using the following formula to evaluate the effect of adding Hpt on stabilizing Hb.

Residual ratio of hemoglobin (%) = (Hemoglobin concentration measured after 7 or 14 days at 37°C/ Hemoglobin concentration measured immediately after mixing hemoglobin with diluent and haptoglobin (initial starting value)) $\times$ 100

The results are listed in Tables 7 to 9.

[Table 7]

| | Hpt | Amount of addition (Unit) | 0 | 0.125 | 0.25 | 0.5 | 1 |
|---|---|---|---|---|---|---|---|
| | | Concentration (pmol/mL) | 0 | 0.775 | 1.55 | 3.1 | 6.2 |
| Porcine Hpt (without enzyme treatment) | Ratio to no Hpt addition (%) | | 100 | 97 | 89 | 81 | 65 |
| Porcine dHpt (ENGase) | | | 100 | 98 | 96 | 94 | 88 |
| Porcine dHpt (PNGase) | | | 100 | 97 | 95 | 89 | 78 |

| | Hpt | Amount of addition (Unit) | 0 | 0.125 | 0.25 | 0.5 | 1 |
|---|---|---|---|---|---|---|---|
| | | Concentration (pmol/mL) | 0 | 0.775 | 1.55 | 3.1 | 6.2 |
| Porcine Hpt (without enzyme treatment) | Residual Hb ratio (%) | Storage 0 days | 100 | 100 | 100 | 100 | 100 |
| Porcine dHpt (ENGase) | | | 100 | 100 | 100 | 100 | 100 |
| Porcine dHpt (PNGase) | | | 100 | 100 | 100 | 100 | 100 |
| Porcine Hpt (without enzyme treatment) | | Storage 7 days | 40 | 40 | 41 | 49 | 79 |
| Porcine dHpt (ENGase) | | | 43 | 45 | 46 | 55 | 86 |
| Porcine dHpt (PNGase) | | | 41 | 41 | 45 | 58 | 88 |
| Porcine Hpt (without enzyme treatment) | | Storage 14 days | 7 | 22 | 32 | 48 | 79 |
| Porcine dHpt (ENGase) | | | 15 | 24 | 33 | 54 | 88 |
| Porcine dHpt (PNGase) | | | 11 | 23 | 34 | 58 | 87 |

[Table 8]

| | Hpt | Amount of addition (Unit) | 0 | 0.125 | 0.25 | 0.5 | 1 |
|---|---|---|---|---|---|---|---|
| | | Concentration (pmol/mL) | 0 | 0.775 | 1.55 | 3.1 | 6.2 |
| Equine Hpt (without enzyme treatment) | Ratio to no Hpt addition (%) | | 100 | 94 | 90 | 74 | 50 |
| Equine dHpt (ENGase) | | | 100 | 101 | 101 | 106 | 93 |
| Equine dHpt (PNGase) | | | 100 | 97 | 92 | 88 | 68 |

| | Hpt | Amount of addition (Unit) | 0 | 0.125 | 0.25 | 0.5 | 1 |
|---|---|---|---|---|---|---|---|
| | | Concentration (pmol/mL) | 0 | 0.775 | 1.55 | 3.1 | 6.2 |
| Equine Hpt (without enzyme treatment) | Residual Hb ratio (%) | Storage 0 days | 100 | 100 | 100 | 100 | 100 |
| Equine dHpt (ENGase) | | | 100 | 100 | 100 | 100 | 100 |
| Equine dHpt (PNGase) | | | 100 | 100 | 100 | 100 | 100 |
| Equine Hpt (without enzyme treatment) | | Storage 7 days | 43 | 46 | 48 | 63 | 102 |
| Equine dHpt (ENGase) | | | 46 | 47 | 52 | 51 | 97 |
| Equine dHpt (PNGase) | | | 42 | 49 | 56 | 71 | 96 |
| Equine Hpt (without enzyme treatment) | | Storage 14 days | 12 | 18 | 28 | 57 | 100 |
| Equine dHpt (ENGase) | | | 13 | 25 | 34 | 34 | 99 |
| Equine dHpt (PNGase) | | | 15 | 28 | 41 | 67 | 101 |

[Table 9]

| | Hpt | Amount of addition (Unit) | 0 | 0.125 | 0.25 | 0.5 | 1 | 2 |
|---|---|---|---|---|---|---|---|---|
| | | Concentration (pmol/mL) | 0 | 0.775 | 1.55 | 3.1 | 6.2 | 12.4 |
| Human Hpt (without enzyme treatment) | Ratio to no Hpt addition (%) | | 100 | 94 | 91 | 85 | 72 | 39 |
| Human dHpt (ENGase) | | | 100 | 98 | 98 | 93 | 87 | 65 |
| Human dHpt (PNGase) | | | 100 | 99 | 94 | 93 | 82 | 47 |

| | Hpt | Amount of addition (Unit) | 0 | 0.125 | 0.25 | 0.5 | 1 | 2 |
|---|---|---|---|---|---|---|---|---|
| | | Concentration (pmol/mL) | 0 | 0.775 | 1.55 | 3.1 | 6.2 | 12.4 |

(continued)

| | | | | | | | | |
|---|---|---|---|---|---|---|---|---|
| Human Hpt (without enzyme treatment) | Residual Hb ratio (%) | Storage 0 days | 100 | 100 | 100 | 100 | 100 | 100 |
| Human dHpt (ENGase) | | | 100 | 100 | 100 | 100 | 100 | 100 |
| Human dHpt (PNGase) | | | 100 | 100 | 100 | 100 | 100 | 100 |
| Human Hpt (without enzyme treatment) | | Storage 7 days | 42 | 45 | 50 | 57 | 85 | 98 |
| Human dHpt (ENGase) | | | 43 | 48 | 51 | 57 | 80 | 101 |
| Human dHpt (PNGase) | | | 44 | 48 | 55 | 62 | 94 | 100 |
| Human Hpt (without enzyme treatment) | | Storage 14 days | 15 | 22 | 30 | 44 | 87 | 100 |
| Human dHpt (ENGase) | | | 13 | 22 | 31 | 44 | 83 | 100 |
| Human dHpt (PNGase) | | | 16 | 23 | 35 | 50 | 95 | 101 |

As listed in A of each of Tables 7 to 9, when comparing the initial starting values with the initial starting values of the samples with no Hpt added, the Hb measurement value decreased by adding Hpt that had not been deglycosylated, and the degree of decrease in the measurement value increased as the amount of Hpt added increased.

In contrast, ENGase-treated or PNGase-treated Hpt suppressed the decrease in the Hb measurement value compared to non-deglycosylated Hpt, with ENGase-treated Hpt exhibiting a particularly high suppression effect.

The above tendency was commonly observed in all the porcine Hpt, equine Hpt, and human Hpt.

As listed in B of each of Tables 7 to 9, ENGase-treated and PNGase-treated Hpt stabilized Hb in an Hpt concentration-dependent manner to the same extent as non-deglycosylated Hpt for all the porcine Hpt, equine Hpt, and human Hpt.

The residual Hb ratio in samples containing 1 Unit of Hpt (i.e., 500 $\mu$L samples containing 3.1 pmol of Hpt and Hb) when stored at 37°C for 7 days was 70% or more.

The above shows that deglycosylated Hpt can suppress change in the Hb measurement values while maintaining the Hb stabilizing effect.

Testing Example 6: Hb stability test (with stool)

(1) Sample preparation

An 80 $\mu$g/mL purified human Hb solution (available from EIKEN CHEMICAL CO., LTD.) was diluted with dilution solution (0.1% BSA/0.9% NaCl/0.1% NaN$_3$/50mM HEPES, pH 7.0) to prepare a 1200 ng/mL Hb solution.

In addition, original solutions of purified porcine Hpt (without deglycosylation), ENGase-treated porcine Hpt, and PNGase-treated porcine Hpt (Hpt concentration (molar equivalent to human Hb) for each had been calculated) were diluted with the above dilution solution to prepare Hpt solutions.

Furthermore, human feces was dissolved in the above dilution solution to prepare a 20% (wt/vol) stool solution.

A sample was prepared by mixing 250 $\mu$L of the above Hb solution, 237.5 $\mu$L of Hpt solution, and 12.5 $\mu$L of 20% stool solution. The obtained sample is 500 $\mu$L and contains 300 ng (4.65 pmol) of Hb, 4.65 pmol of each type of Hpt, and 0.5% feces.

The amount of Hpt in the above sample (4.65 pmol) was defined as 1 Unit in this test, and samples were prepared with 1.0, 0.5, and 0.25 Units of Hpt added in this test.

Samples were also prepared for equine Hpt and human Hpt in the same manner as for porcine Hpt.

Furthermore, samples with no Hpt added but with dilution solution added (no Hpt added) were also prepared as controls.

(2) Measurement

The Hb values of these samples were measured using an Hb measurement reagent (OC-Hemodia(R) Auto III 'Eiken' available from EIKEN CHEMICAL CO., LTD.) and a fully automated fecal occult blood immunochemical analyzer (OC Sensor DIANA available from EIKEN CHEMICAL CO., LTD.). The measured values were used as the initial starting values.

The samples with the initial starting values measured were then stored at 37°C for 7 and 14 days, and the Hb values were measured again.

The initial starting values were compared to the initial starting values of samples with no Hpt added, and the change in Hb measurement value due to the addition of Hpt was evaluated.

In addition, the measurement values after 7 and 14 days of storage were compared to respective initial starting values, and the effect of adding Hpt on stabilizing Hb was evaluated.

The results are listed in Tables 10 to 12.

[Table 10]

| | Hpt | Amount of addition (Unit) | 0 | 0.25 | 0.5 | 1 |
|---|---|---|---|---|---|---|
| | | Concentration (pmol/mL) | 0 | 2.325 | 4.65 | 9.3 |
| Porcine Hpt (without enzyme treatment) | Ratio to no Hpt addition (%) | | 100 | 93 | 84 | 69 |
| Porcine dHpt (ENGase) | | | 100 | 100 | 98 | 94 |
| Porcine dHpt (PNGase) | | | 100 | 95 | 91 | 81 |

| | Hpt | Amount of addition (Unit) | 0 | 0.25 | 0.5 | 1 |
|---|---|---|---|---|---|---|
| | | Concentration (pmol/mL) | 0 | 2.325 | 4.65 | 9.3 |
| Porcine Hpt (without enzyme treatment) | Residual Hb ratio (%) | Storage 0 days | 100 | 100 | 100 | 100 |
| Porcine dHpt (ENGase) | | | 100 | 100 | 100 | 100 |
| Porcine dHpt (PNGase) | | | 100 | 100 | 100 | 100 |
| Porcine Hpt (without enzyme treatment) | | Storage 7 days | 6 | 19 | 34 | 75 |
| Porcine dHpt (ENGase) | | | 6 | 18 | 30 | 57 |
| Porcine dHpt (PNGase) | | | 6 | 19 | 34 | 69 |
| Porcine Hpt (without enzyme treatment) | | Storage 14 days | 1 | 16 | 34 | 78 |
| Porcine dHpt (ENGase) | | | 1 | 16 | 29 | 56 |
| Porcine dHpt (PNGase) | | | 1 | 16 | 32 | 67 |

[Table 11]

| | Hpt | Amount of addition (Unit) | 0 | 0.25 | 0.5 | 1 |
|---|---|---|---|---|---|---|
| | | Concentration (pmol/mL) | 0 | 2.325 | 4.65 | 9.3 |
| Equine Hpt (without enzyme treatment) | Ratio to no Hpt addition (%) | | 100 | 93 | 85 | 67 |
| Equine dHpt (ENGase) | | | 100 | 100 | 101 | 96 |
| Equine dHpt (PNGase) | | | 100 | 98 | 97 | 92 |

(continued)

| | Hpt | Amount of addition (Unit) | 0 | 0.25 | 0.5 | 1 |
|---|---|---|---|---|---|---|
| | | Concentration (pmol/mL) | 0 | 2.325 | 4.65 | 9.3 |
| Equine Hpt (without enzyme treatment) | | Storage 0 days | 100 | 100 | 100 | 100 |
| Equine dHpt (ENGase) | | Storage 0 days | 100 | 100 | 100 | 100 |
| Equine dHpt (PNGase) | | Storage 0 days | 100 | 100 | 100 | 100 |
| Equine Hpt (without enzyme treatment) | Residual Hb ratio (%) | Storage 7 days | 6 | 21 | 39 | 93 |
| Equine dHpt (ENGase) | | Storage 7 days | 6 | 22 | 36 | 74 |
| Equine dHpt (PNGase) | | Storage 7 days | 6 | 21 | 36 | 68 |
| Equine Hpt (without enzyme treatment) | | Storage 14 days | 1 | 18 | 38 | 95 |
| Equine dHpt (ENGase) | | Storage 14 days | 1 | 19 | 34 | 73 |
| Equine dHpt (PNGase) | | Storage 14 days | 1 | 17 | 32 | 66 |

[Table 12]

| | Hpt | Amount of addition (Unit) | 0 | 0.25 | 0.5 | 1 |
|---|---|---|---|---|---|---|
| | | Concentration (pmol/mL) | 0 | 2.325 | 4.65 | 9.3 |
| Human Hpt (without enzyme treatment) | | Ratio to no Hpt addition (%) | 100 | 95 | 88 | 75 |
| Human dHpt (ENGase) | | | 100 | 98 | 94 | 86 |
| Human dHpt (PNGase) | | | 100 | 99 | 93 | 87 |
| | Hpt | Amount of addition (Unit) | 0 | 0.25 | 0.5 | 1 |
| | | Concentration (pmol/mL) | 0 | 2.325 | 4.65 | 9.3 |
| Human Hpt (without enzyme treatment) | | Storage 0 days | 100 | 100 | 100 | 100 |
| Human dHpt (ENGase) | | | 100 | 100 | 100 | 100 |
| Human dHpt (PNGase) | | | 100 | 100 | 100 | 100 |
| Human Hpt (without enzyme treatment) | Residual Hb ratio (%) | Storage 7 days | 6 | 23 | 44 | 97 |
| Human dHpt (ENGase) | | Storage 7 days | 6 | 23 | 41 | 84 |
| Human dHpt (PNGase) | | Storage 7 days | 6 | 23 | 43 | 82 |
| Human Hpt (without enzyme treatment) | | Storage 14 days | 1 | 21 | 41 | 96 |
| Human dHpt (ENGase) | | Storage 14 days | 1 | 19 | 40 | 84 |
| Human dHpt (PNGase) | | Storage 14 days | 1 | 18 | 40 | 80 |

As listed in Tables 10 to 12, even when the measurement sample contained feces, a similar tendency was observed as in the case of no feces.

In other words, the addition of non-deglycosylated Hpt reduced the Hb measurement value (initial starting value) in a concentration-dependent manner, but this reduction was suppressed in the case of ENGase-treated or PNGase-treated Hpt.

Furthermore, ENGase-treated and PNGase-treated Hpt stabilized Hb in an Hpt concentration-dependent manner.

The above tendency was commonly confirmed in all the porcine Hpt, equine Hpt, and human Hpt.

**Claims**

1.  An immunological measurement method for hemoglobin, comprising

    a step of performing the immunological measurement using an anti-hemoglobin antibody under coexistence of the hemoglobin and haptoglobin,
    the haptoglobin having a characteristic (1) below:

    (1) at least a portion of an N-linked glycan is deleted.

2.  The immunological measurement method according to claim 1, wherein the haptoglobin further has a characteristic (2) below:
    (2) when 3.1 pmol of hemoglobin is stored at 37°C for 7 days in 500 μL of a buffer solution containing 3.1 pmol of the haptoglobin, a residual ratio of the hemoglobin is 70% or more.

3.  The immunological measurement method according to claim 1 or 2, wherein the characteristic (1) is a characteristic (1a) below:
    (1a) a difference between a molecular weight of a β-chain in the haptoglobin treated with peptide-N-glycosidase (PNGase) and a molecular weight of a β-chain in an untreated haptoglobin is 6,500 or less.

4.  The immunological measurement method according to claim 1 or 2, wherein the haptoglobin has no N-linked glycan.

5.  The immunological measurement method according to claim 1 or 2, wherein the N-linked glycan present in the haptoglobin from which at least a portion of the N-linked glycan is deleted is one N-acetylglucosamine residue or a glycan in which one fucose residue is bound to one N-acetylglucosamine residue.

6.  The immunological measurement method according to claim 1 or 2, wherein the hemoglobin is contained in feces, blood, serum, plasma, urine, sputum, or saliva.

7.  The immunological measurement method according to claim 1 or 2, wherein at least one type of the anti-hemoglobin antibody is supported on an insoluble carrier.

8.  The immunological measurement method according to claim 7, wherein the insoluble carrier is an insoluble particle.

9.  The immunological measurement method according to claim 1 or 2, wherein the immunological measurement method is an immune agglutination method.

10. A method for suppressing, in immunological measurement of hemoglobin, change in measurement values of the hemoglobin, comprising

    a step of performing the immunological measurement using an anti-hemoglobin antibody under coexistence of the hemoglobin and haptoglobin,
    the method using as the haptoglobin a haptoglobin having a characteristic (1) below:

    (1) at least a portion of an N-linked glycan is deleted.

11. The method for suppressing change in measurement values of hemoglobin according to claim 10, wherein the haptoglobin further has a characteristic (2) below:
    (2) when 3.1 pmol of hemoglobin is stored at 37°C for 7 days in 500 μL of a buffer solution containing 3.1 pmol of the haptoglobin, a residual ratio of the hemoglobin is 70% or more.

12. A method for stabilizing hemoglobin, comprising a step of allowing hemoglobin to coexist with haptoglobin having a characteristic (1) below:

    (1) at least a portion of an N-linked glycan is deleted.

13. The method for stabilizing hemoglobin according to claim 12, wherein the haptoglobin further has a characteristic (2) below:

(2) when 3.1 pmol of hemoglobin is stored at 37°C for 7 days in 500 μL of a buffer solution containing 3.1 pmol of the haptoglobin, a residual ratio of the hemoglobin is 70% or more.

14. The method for stabilizing hemoglobin according to claim 12 or 13, wherein the haptoglobin has no N-linked glycan.

15. The method for stabilizing hemoglobin according to claim 12 or 13, wherein the N-linked glycan present in the haptoglobin from which at least a portion of the N-linked glycan is deleted is one N-acetylglucosamine residue or a glycan in which one fucose residue is bound to one N-acetylglucosamine residue.

16. A solution for stabilizing hemoglobin, comprising haptoglobin having a characteristic (1) below:

    (1) at least a portion of an N-linked glycan is deleted.

17. The solution for stabilizing hemoglobin according to claim 16, wherein the haptoglobin further has a characteristic (2) below:
    (2) when 3.1 pmol of hemoglobin is stored at 37°C for 7 days in 500 μL of a buffer solution containing 3.1 pmol of the haptoglobin, a residual ratio of the hemoglobin is 70% or more.

18. The solution for stabilizing hemoglobin according to claim 16 or 17, wherein the characteristic (1) is a characteristic (1a) below:
    (1a) a difference between a molecular weight of a β-chain in the haptoglobin treated with peptide-N-glycosidase (PNGase) and a molecular weight of a β-chain in an untreated haptoglobin is 6,500 or less.

19. The solution for stabilizing hemoglobin according to claim 16 or 17, wherein the haptoglobin has no N-linked glycan.

20. The solution for stabilizing hemoglobin according to claim 16 or 17, wherein the haptoglobin is haptoglobin that is naturally-derived and treated with peptide-N-glycosidase (PNGase).

21. The solution for stabilizing hemoglobin according to claim 16 or 17, wherein the N-linked glycan present in the haptoglobin from which at least a portion of the N-linked glycan is deleted is one N-acetylglucosamine residue or a glycan in which one fucose residue is bound to one N-acetylglucosamine residue.

22. The solution for stabilizing hemoglobin according to claim 16 or 17, wherein the haptoglobin is haptoglobin that is naturally-derived and treated with endo-β-N-acetylglucosaminidase (ENGase).

23. The solution for stabilizing hemoglobin according to claim 16 or 17, wherein the hemoglobin is contained in feces, blood, serum, plasma, urine, sputum, or saliva.

24. A hemoglobin measurement kit for immunologically measuring hemoglobin, comprising:

    the solution for stabilizing hemoglobin according to claim 16 or 17; and
    a reagent containing an anti-hemoglobin antibody.

25. The hemoglobin measurement kit according to claim 24, wherein at least one type of the anti-hemoglobin antibody is supported on an insoluble carrier.

26. The hemoglobin measurement kit according to claim 25, wherein the insoluble carrier is an insoluble particle.

27. The hemoglobin measurement kit according to claim 24, wherein the reagent is a reagent for an immune agglutination method.

28. Haptoglobin having characteristics (1) and (2) below:

    (1) at least a portion of an N-linked glycan is deleted; and
    (2) when 3.1 pmol of hemoglobin is stored at 37°C for 7 days in 500 μL of a buffer solution containing 3.1 pmol of the haptoglobin, a residual ratio of the hemoglobin is 70% or more.

29. The haptoglobin according to claim 28, wherein the characteristic (1) is a characteristic (1a) below:

(1a) a difference between a molecular weight of a β-chain in the haptoglobin treated with peptide-N-glycosidase (PNGase) and a molecular weight of a β-chain in an untreated haptoglobin is 6,500 or less.

30. The haptoglobin according to claim 28 or 29, wherein the haptoglobin has no N-linked glycan.

31. The haptoglobin according to claim 28 or 29, wherein the haptoglobin is haptoglobin that is naturally-derived and treated with peptide-N-glycosidase (PNGase).

32. The haptoglobin according to claim 28 or 29, wherein the N-linked glycan present in the haptoglobin from which at least a portion of the N-linked glycan is deleted is one N-acetylglucosamine residue or a glycan in which one fucose residue is bound to one N-acetylglucosamine residue.

33. The haptoglobin according to claim 28 or 29, wherein the haptoglobin is haptoglobin that is naturally-derived and treated with endo-β-N-acetylglucosaminidase (ENGase).

34. Haptoglobin having an N-linked glycan, the glycan of the N-linked glycan being one N-acetylglucosamine residue or only a glycan in which one fucose residue is bound to one N-acetylglucosamine residue.

35. A method for producing haptoglobin, comprising a step of subjecting naturally-derived haptoglobin to a glycosidase treatment, and satisfying (a) and (b) below:

(a) the naturally-derived haptoglobin is not subjected to a heat shock of 90°C or higher prior to the glycosidase treatment; and
(b) SDS is not allowed to coexist during the glycosidase treatment.

36. The method for producing haptoglobin according to claim 35, wherein the glycosidase is peptide-N-glycosidase (PNGase).

37. The method for producing haptoglobin according to claim 36, wherein the peptide-N-glycosidase is PNGase F.

38. The method for producing haptoglobin according to claim 35, wherein the glycosidase is endo-β-N-acetylglucosaminidase (ENGase).

39. The method for producing haptoglobin according to claim 38, wherein the endo-β-N-acetylglucosaminidase is Endo F2.

[FIG. 1]

| | : N-acetylglucosamine (GlcNAc) | | : Galactose (Gal) |
| --- | --- | --- | --- |
| | : Mannose (Man) | | : Fucose (Fuc) |
| | | | : Sialic acid |

[FIG. 2]

SEQ ID NO: 1

```
MSALGAVIAL LLWGQLFAVD SGNDVTDIAD DGCPKPPEIA HGYVEHSVRY QCKNYYKLRT 60
EGDGVYTLND KKQWINKAVG DKLPECEADD GCPKPPEIAH GYVEHSVRYQ CKNYYKLRTE 120
GDGVYTLNNE KQWINKAVGD KLPECEAVCG KPKNPANPVQ RILGGHLDAK GSFPWQAKMV 180
SHHNLTTGAT LINEQWLLTT AKNLFLNHSE NATAKDIAPT LTLYVGKKQL VEIEKVVLHP 240
NYSQVDIGLI KLKQKVSVNE RVMPICLPSK DYAEVGRVGY VSGWGRNANF KFTDHLKYVM 300
LPVADQDQCI RHYEGSTVPE KKTPKSPVGV QPILNEHTFC AGMSKYQEDT CYGDAGSAFA 360
VHDLEEDTWY ATGILSFDKS CAVAEYGVYV KVTSIQDWVQ KTIAEN              406
```

[FIG. 3]

SEQ ID NO: 2

```
MSALGAVIAL LLWGQLFAVD SGNDVTDIAD DGCPKPPEIA HGYVEHSVRY QCKNYYKLRT 60
EGDGVYTLNN EKQWINKAVG DKLPECEAVC GKPKNPANPV QRILGGHLDA KGSFPWQAKM 120
VSHHNLTTGA TLINEQWLLT TAKNLFLNHS ENATAKDIAP TLTLYVGKKQ LVEIEKVVLH 180
PNYSQVDIGL IKLKQKVSVN ERVMPICLPS KDYAEVGRVG YVSGWGRNAN FKFTDHLKYV 240
MLPVADQDQC IRHYEGSTVP EKKTPKSPVG VQPILNEHTF CAGMSKYQED TCYGDAGSAF 300
AVHDLEEDTW YATGILSFDK SCAVAEYGVY VKVTSIQDWV QKTIAEN 347
```

[FIG. 4]

SEQ ID NO: 3

```
MSALGAVVAL LLWGQLFAVD TGNEATDFTD DSCPKPPEIP NGYVEHLVRY QCKNYYRLRS 60
EGDGVYALNS EKQWVNKASG TKLPECEAVC GKPKNPVDQV QRIIGGLLDA KGSFPWQAKL 120
VSRHNLTTGA TLISEQWLLT TAQNLFLNHT PDAKPKDIAP TLKLYVGRKQ PVEIEKVVFH 180
PDYQEVDIGL IKLKEKVPVG ERVMPICLPS KDYAQVGRVG YVSGWGRNAN FNFTELLKYV 240
TLPVADQDTC VKHYEGSTVP EKKTNRSSVG VQPILNEHTF CAGLSKFQED TCYGDAGSAF 300
VIHDEEDDTW YAAGILSFDK SCAVAEYGVY VKVPSILDWV QKTIAEN 347
```

[FIG. 5]

SEQ ID NO: 4

```
MRALGAVVAL LLCGQLFAAE TGNEATDATD DSCPKPPEIP KGYVEHMVRY HCQTYYKLRT 60
AGDGVYTLDS NKQWTNKVTG EKLPECEAVC GKPKNPVDQV QRIMGGSLDA KGSFPWQAKM 120
ISHHNLTSGA TLINEQWLLT TAKNLRLGHK NDTKAKDIAP TLRLYVGKKQ EVEIEKVIFH 180
PDNSTVDIGL IKLKQKVPVN ERVMPICLPS KDYVNVGLVG YVSGWGRNAN LNFTEHLKYV 240
MLPVADQEKC VQYYEGSTVP EKKTPKSPVG VQPILNEHTF CAGLSKYQED TCYGDAGSAF 300
AVHDKDDDTW YAAGILSFDK SCRTAEYGVY VRVTSILDWI QTTIADN 347
```

[FIG. 6]

**Measurement of free-Hb/Hb-Hp complex mixture solution**

X Colorimetric assay

O Latex reagent

[FIG. 7]

[FIG. 8]

(a)
Equine Hpt
(without enzyme
treatment)

(b)
Equine Hpt
(ENGase treatment)

(c)
Equine Hpt
(PNGase treatment)

[FIG. 9]

(a)
Equine Hpt
(without enzyme treatment)

(b)
Equine Hpt
(without enzyme treatment
→ PNGase treatment)

(c)
Equine dHpt
(ENGase treatment)

(d)
Equine dHpt
(ENGase treatment
→ PNGase treatment)

[FIG. 10]

Relationship between free-Hb concentration and Hp addition amount in free-Hb specific measurement

## INTERNATIONAL SEARCH REPORT

| International application No. |
| --- |
| **PCT/JP2024/007134** |

| A. | CLASSIFICATION OF SUBJECT MATTER |
| --- | --- |

*G01N 33/53*(2006.01)i; *C07K 14/435*(2006.01)i; *C12P 21/02*(2006.01)i; *G01N 33/72*(2006.01)i; *G01N 33/543*(2006.01)i
FI:   G01N33/53 D; G01N33/72 A; G01N33/543 581A; C12P21/02 Z; C07K14/435 ZNA

According to International Patent Classification (IPC) or to both national classification and IPC

| B. | FIELDS SEARCHED |
| --- | --- |

Minimum documentation searched (classification system followed by classification symbols)

G01N33/53; C07K14/435; C12P21/02; G01N33/72; G01N33/543

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 10-132824 A (EIKEN CHEMICAL) 22 May 1998 (1998-05-22) claim 1 | 1-39 |
| A | WO 2020/066722 A1 (EIKEN CHEMICAL) 02 April 2020 (2020-04-02) paragraphs [0004]-[0005] | 1-39 |

☐ Further documents are listed in the continuation of Box C.       ☑ See patent family annex.

| | |
| --- | --- |
| * | Special categories of cited documents: |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 May 2024** | **14 May 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**

International application No.

**PCT/JP2024/007134**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/007134**

| Patent document cited in search report | | | Publication date (day/month/year) | Patent family member(s) | | | Publication date (day/month/year) |
|---|---|---|---|---|---|---|---|
| JP | 10-132824 | A | 22 May 1998 | (Family: none) | | | |
| WO | 2020/066722 | A1 | 02 April 2020 | US | 2022/0043008 | A1 | |
| | | | | paragraphs [0004]-[0005] | | | |
| | | | | EP | 3859338 | A1 | |
| | | | | CN | 112752974 | A | |
| | | | | KR | 10-2021-0064272 | A | |
| | | | | JP | 2022-33286 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10132824 A
- WO 2011058958 A

- WO 2020066722 A